# EUROPEAN PATENT SPECIFICATION

(11) **EP 2 734 227 B1**
(45) Date of publication and mention of the grant of the patent: **19.09.2018**
(21) Application number: 12753158.0
(22) Date of filing: 23.07.2012
(51) Int. Cl.: A61K 39/00

(54) **VACCINE**
IMPFSTOFF
VACCIN

(30) Priority: 22.07.2011 GB 201112587
(43) Date of publication of application: 28.05.2014
(73) Proprietor: Moredun Research Institute, Bush Loan Penicuik Midlothian EH26 0PZ (GB)
(72) Inventor: HUNTLEY, John Frederick, Penicuik Midlothian EH26 0PZ (GB); NISBET, Alasdair Justice, Penicuik Midlothian EH26 0PZ (GB)
(74) Representative: Chapman, Paul Gilmour
(86) International application number: PCT/GB2012/000608
(87) International publication number: WO 2013/014408

(56) References cited:
- WO-A2-2016/162672
- HARRY W WRIGHT ET AL: "The testing of antibodies raised against poultry red mite antigens in an in vitro feeding assay; preliminary screen for vaccine candidates", EXPERIMENTAL AND APPLIED ACAROLOGY, KLUWER ACADEMIC PUBLISHERS, DO, vol. 48, no. 1-2, 29 January 2009 (2009-01-29), pages 81-91, XP019669442, ISSN: 1572-9702
- BARTLEY K ET AL: "Histamine Release Factor from Dermanyssus gallinae (De Geer): Characterization and in vitro assessment as a protective antigen", INTERNATIONAL JOURNAL OF PARASITOLOGY, PERGAMON PRESS, GB, vol. 39, no. 4, 1 March 2009 (2009-03-01), pages 447-456, XP025926526, ISSN: 0020-7519, DOI: 10.1016/J.IJPARA.2008.09.006 [retrieved on 2009-01-31]
- HARRINGTON D ET AL: "Characterization of the immune response of domestic fowl following immunization with proteins extracted from Dermanyssus gallinae", VETERINARY PARASITOLOGY, ELSEVIER SCIENCE, AMSTERDAM, NL, vol. 160, no. 3-4, 23 March 2009 (2009-03-23), pages 285-294, XP025995308, ISSN: 0304-4017, DOI: 10.1016/J.VETPAR.2008.11.004 [retrieved on 2008-11-13]
- ARKLE SAM ET AL: "Immunological control of the poultry red mite.", ANNALS OF THE NEW YORK ACADEMY OF SCIENCES DEC 2008, vol. 1149, December 2008 (2008-12), pages 36-40, XP002692546, ISSN: 1749-6632
- DATABASE UniProt [Online] 10 February 2009 (2009-02-10), "SubName: Full=Vitellogenin, putative;", XP002692547, retrieved from EBI accession no. UNIPROT:B7QJ67 Database accession no. B7QJ67
- DATABASE UniProt [Online] 10 February 2009 (2009-02-10), "SubName: Full=Vitellogenin;", XP002692548, retrieved from EBI accession no. UNIPROT:B6ZIV7 Database accession no. B6ZIV7
- Mcguire: "Quality Control Analytical Methods: The Quality of Sterility Testing", International Journal of Pharmaceutical Compounding, 1 January 2007 (2007-01-01), XP055291072, Retrieved from the Internet: URL:http://arlok.com/articles/The Quality of Sterility Testing.pdf [retrieved on 2016-07-25]
- KATHRYN BARTLEY ET AL: "Identification and evaluation of vaccine candidate antigens from the poultry red mite (Dermanyssus gallinae)", INTERNATIONAL JOURNAL OF PARASITOLOGY, vol. 45, no. 13, 1 November 2015 (2015-11-01), pages 819-830, XP055287501, GB ISSN: 0020-7519, DOI: 10.1016/j.ijpara.2015.07.004

### SEQUENCE LISTING

<110> Moredun Research Institute
<120> poultry vaccine
<130> PG443190WO
<150> GB1112587.9
   <151> 2011-07-22
<160>
<170> Patentin version 3.3
<210> 1
   <211> 1861
   <212> PRT
   <213> Dermanyssus gallinae
<400> 1

Val Trp Leu Phe Val Asn Pro Ser Thr Met Arg Phe Phe Val Leu Pro
1               5                   10                  15

Leu Leu Leu Ala Ala Ala Ala Ser Ala Glu Val Phe His Phe Val Gly
            20                  25                  30

Gln His Gly Gln Gly Ser Thr Val Tyr Gly Val Arg Gly Ala Val Thr
        35                  40                  45

Val Gly Ala His Gln Leu Thr Ala Glu Lys Thr Ala Leu Glu Tyr Asn
    50                  55                  60

Gly Thr Leu Ala Val Glu Gln Ile Arg Glu Gly Glu Phe Leu Thr Lys
65                  70                  75                  80

Phe Thr His Phe Thr Val Gly Lys Tyr Asn Lys Leu Gln Arg Ser Val
                85                  90                  95

Gln Asp Glu Thr Phe Asp Asp Leu Thr Pro Glu Glu Gln Arg Val Val
            100                 105                 110

Lys Thr Leu Arg Glu Pro Ala Val Tyr Glu Pro His Met Gln Arg Pro
        115                 120                 125

Val Arg Phe Phe Val Lys Glu Gly Gln Ile Val Arg Met Glu Ala Glu
    130                 135                 140

Lys Glu His Pro Gln Trp Ser Leu Asn Ile Phe Arg Ser Val Leu Thr
145                 150                 155                 160

Leu Phe Gln Asn Gln Val Ser Lys Pro Ala Thr Leu Ala Val Pro His
                165                 170                 175

Val Glu Tyr Lys Tyr Glu Asp Gly Ile Thr Gly Asn Cys Lys Val Gln
            180                 185                 190

Tyr Glu Val Phe Ser Leu Pro Glu Asp Val Thr Val Gln Gly Val Phe
        195                 200                 205

Asn Leu Thr Lys Thr Lys Asn Tyr Lys Asp Cys Leu Gly Arg Pro Val
    210                 215                 220

Tyr Leu His Leu Lys Asp Thr Gln Arg Gly Cys Ala Gly Val Cys Asp
225                 230                 235                 240

Asn His Arg Pro Glu Asn Phe Leu Ala Gly Tyr Glu Glu Glu Ile Thr
                245                 250                 255

Asp Tyr Glu Leu Lys Pro Thr Pro Gly Cys Pro Val Asn Gln Gln Arg
            260                 265                 270

Lys Asp Thr Leu Val Thr Val Gln Thr Val Thr Lys Tyr Asn Val Ser
        275                 280                 285

Asn Gly Tyr Leu Asp Glu Val Arg Ser Glu His Thr Asp Ile Tyr Arg
    290                 295                 300

Leu Tyr Gly Gly Lys Leu His Val Phe Thr Thr Leu Gln Leu Arg Leu
305                 310                 315                 320

Tyr Gly Val Ala Gly Pro Lys Ile Glu Glu Pro Lys Thr Val Glu Ile
                325                 330                 335

Tyr Lys Thr Leu Gln Leu Arg Leu Pro His Glu Glu Asp Glu Leu Asp
            340                 345                 350

Ile Pro Val Tyr Ala Leu Leu Arg Glu His Thr Thr Gln Gln Gln Tyr
        355                 360                 365

Gly Gln His Phe Gln Lys Tyr Phe Glu Ala Val Val Gln Glu Leu Leu
    370                 375                 380

Gln Leu Lys Asp Thr Gln Lys Gln Gly Gln Pro Glu Lys Gln His Tyr
385                 390                 395                 400

His Ser Thr Ala Tyr Leu Val Glu Leu Val Gln Ala Val Ser Ser Met
                405                 410                 415

Thr Glu Lys Glu Leu Lys Ser Ile Ile Pro Thr Ile Val His Gln Ala
            420                 425                 430

Gln Pro Lys Gln Leu Thr Glu Glu Glu His Val Arg Arg Gln Leu Trp
        435                 440                 445

Val Glu Leu Leu Gly Lys Ala Gly Ser Lys Ser Ala Val Lys Ile Ile
    450                 455                 460

Val Glu Leu Val Lys Gly Lys Leu Leu Thr Pro Thr Glu Ile Arg Arg
465                 470                 475                 480

Val Leu Gln Asp Val Ala Ala Phe Gln Ser Tyr Pro Asp Thr Glu Met
                485                 490                 495

Val Glu Gln Ile Leu Ala Leu Cys Val Lys Glu Gln Gly Leu Thr Ala
            500                 505                 510

Thr Gly Lys Ala Thr Ala Cys Val Ala Ala Gly Lys Val Leu Ser Lys
        515                 520                 525

Ala Cys Asn Ser Lys Val Tyr Gln Leu Ala Gln Lys His Glu Gln His
    530                 535                 540

Lys Lys Thr Ile Asn Gly Lys Tyr Gln Ser Ile Val Gln Met Gln Glu
545                 550                 555                 560

Gln Lys Tyr Thr Pro Glu Thr Glu Pro Glu Ala Glu Glu Tyr Arg Val
                565                 570                 575

Thr Phe Gly His Leu Pro Val Asp Pro Lys Leu Val Cys Thr Pro Glu
            580                 585                 590

Lys Leu Gln Lys Tyr Val Ser Asp Leu Ser His Ala Leu His Gln Ala
        595                 600                 605

Thr Asp Phe Lys His Val Val Ala Tyr Ile Asn Gly Leu Ala His Val
    610                 615                 620

Gln Lys Pro Glu Val Leu Pro Glu Leu Leu Gly Tyr Val Asn Gly Thr
625                 630                 635                 640

Ala Ser Asn Leu Val His Ile His Glu Gln Gly Glu Asp Ile Lys Glu
                645                 650                 655

Ala Val Glu Phe Ala Arg His Val Ala Ile Val Ser Leu Gln His Val
            660                 665                 670

Ala Val Lys Tyr Pro Lys Glu Val Asn Pro Ile Val Arg Val Val Phe
        675                 680                 685

Glu Asn Thr Thr Glu Lys Val Gln Thr Arg Ile Leu Ala Phe Asp Val
    690                 695                 700

Trp Met Asp Thr Gln Pro Ala Gln Trp Glu Val Glu Lys Val Met Gln
705                 710                 715                 720

Ile Ala Asn Lys Asp Ser Ser Leu Glu Leu Thr His Tyr Val Tyr Thr
                725                 730                 735

Ala Leu Lys Thr Ala Met Lys Ala Glu Glu Pro Cys Tyr Gln Leu Leu
            740                 745                 750

Ala Gln Arg Val Arg Ala Ala Trp Thr Gln Leu Arg Pro Phe Asp Leu
        755                 760                 765

Gly Ser Glu Phe Ser His Leu Arg Ser Lys Phe Tyr Tyr Asp Thr Val
    770                 775                 780

Glu Asn Tyr Gly Ile Arg Gly Val Trp Lys Val Ile Ala Ser Asn Thr
785                 790                 795                 800

Thr Ile Leu Pro Phe Tyr Thr Glu Ala Lys Val Asn Gln Val Arg Gly
                805                 810                 815

Pro Tyr Lys Thr Thr Leu Phe Gly Ala Lys Leu Leu Val Lys Gly Gly
            820                 825                 830

Asp Lys Val Leu Glu Glu Leu Val Gly Lys Asp Gly Leu Leu Glu Arg
        835                 840                 845

Ile Ala Tyr Ala Leu Val Gly Gln Ile Lys Thr Gly Pro Arg Gln Gln
    850                 855                 860

Asn Thr Glu Gln Leu Leu Lys Asp Ile Ala Gln Gly Met Gly Leu Lys
865                 870                 875                 880

Arg Glu Lys Asp Glu Thr Pro Lys Ala Val Leu Phe Trp Lys Leu Phe
                885                 890                 895

Ser Gly Asp Ala Val Ile Pro Leu Asp Ser His Tyr Ile Asn Glu Leu
            900                 905                 910

Lys Gln Glu Leu Leu Gln Thr Val Thr Lys Phe Gly Lys Asp Gly Val
        915                 920                 925

Thr Gly His Ile Val Arg Val Leu Val Pro Thr Lys Ala Phe His Val
    930                 935                 940

Glu Pro Ser Thr Ile Gly Leu Pro Ile Val His Ser Thr Ile His Pro
945                 950                 955                 960

Val Val Leu Ser Val Arg Tyr Glu Asn Ile Lys Ile His Tyr Gly Asn
                965                 970                 975

Gln Glu Ser Arg Val Ala Pro Lys Thr Leu Glu Ile Ser Gly Thr Val
            980                 985                 990

Gln Pro Thr Ile Leu Ser Phe Arg  Gln Ser Arg Val Phe  Val Ser Asp
        995                 1000                1005

Lys Val Gly Gln Lys Asn Pro Thr Val Lys Thr Thr Asp Ile Lys
    1010                1015                1020

Glu Phe Asn Val Arg Leu Ala Phe Arg Val Val Tyr Glu His Thr
    1025                1030                1035

Pro Lys Arg Phe Arg Val His Val Lys Pro Val Phe Asp Arg Val
    1040                1045                1050

Phe His Ser Gly His Cys Thr Glu Leu Lys Leu Glu Ser Ala Val
    1055                1060                1065

Leu Leu Lys Glu Glu Leu Ala Ala Lys Thr Val Glu Tyr Asp Lys
    1070                1075                1080

Cys Ile Lys Ser Leu Tyr Gln Pro Ile Arg Arg Asn His Gln Ile
    1085                1090                1095

Ala Gly Glu Trp Ser Gly Met Met Leu Arg Leu Thr Gly Glu Ser
    1100                1105                1110

His Gln Pro Trp Ser Gly Leu Pro Met Phe Ala Pro Ser Val Val
    1115                1120                1125

Ser Ser Glu Gly Ile Leu Gly Ala Ile Ile Asn Arg Leu Ser Asn
    1130                1135                1140

Lys Gly Met Lys His His Thr Val Ser Leu Tyr Leu Glu Thr Asn
    1145                1150                1155

Asn Gln Gln Pro Ile Thr Glu Trp Val Ala Thr Ile Asp Val Asp
    1160                1165                1170

Ser Asn Val Glu Arg Leu Ala Lys Val Pro Leu Ser Gln Gln Ile
    1175                1180                1185

Thr Lys Val Gln Lys Leu Lys Val Gln Tyr Ala Asn Arg Ala Gln
    1190                1195                1200

Pro Leu Tyr Pro Glu Leu Glu Pro Leu Val Arg Lys Val Glu Ser
    1205                1210                1215

Leu Leu Glu Lys Phe Glu Thr Leu Asp Glu Thr Thr Val Glu Lys
    1220                1225                1230

Leu Met Leu Val Lys Ile Glu Gly Leu Tyr Gln Gly Gln Pro Lys
    1235                1240                1245

Ser Thr Leu Lys Ile Ala Met Lys Lys Ile Tyr Asn Leu Glu Lys
    1250                1255                1260

Thr Glu Gln Gln Tyr Ala Leu Ala Ala Met His Gln Glu Ser His
    1265                1270                1275

Lys Gly Leu Glu Leu Ser Thr Asn Val Ser Tyr Pro Lys Ile Gly
    1280                1285                1290

Ser Pro Phe Arg Tyr Asp Pro Thr Phe Tyr Ala Glu Asp Glu Arg
    1295                1300                1305

Met Asn Gly Thr Leu Ile Val Lys Leu Gln Ser Pro Gln Glu Gln
    1310                1315                1320

Val Phe His Val Lys Phe Gln Ala Thr Lys Ser Glu Glu Gln Leu
    1325                1330                1335

Lys Glu Thr Glu Tyr Glu Trp Phe Glu Val Arg Cys Leu Ala Glu
    1340                1345                1350

Gln Lys Ala Gly Lys Ile Met Thr Asp Ala Cys Arg Lys Ala Val
    1355                1360                1365

Leu Lys Asp Asn Ser Leu Asp Gln Leu Lys Ile Ala Val Thr Val
    1370                1375                1380

Pro Arg Asn Val His Pro Lys Ile Gln Thr Leu Ala Tyr Lys Thr
    1385                1390                1395

Leu Asp Leu Met Lys Tyr Met Trp Tyr Pro Lys Met Gln Thr Glu
    1400                1405                1410

Val Ala Gly Leu Lys Gln Arg Glu Val Leu Gln Ala Leu Gln His
    1415                1420                1425

Thr Glu Arg Glu Val Arg Ile Ser Val Asn Ala Thr Arg Glu Ser
    1430                1435                1440

Leu Trp His Leu Leu Tyr Asp Val Arg Val Glu Met Pro Phe Glu
    1445                1450                1455

Asn Val Thr Phe Ser Lys Val Asn Ile Pro Gly Val Arg Pro Ala
    1460                1465                1470

His Met Gln Leu Thr Thr Lys Glu Gln Leu Glu His Val Tyr Tyr
    1475                1480                1485

Arg Gly Gln Lys Asp Asn Val Cys Val Leu Gly Asp Lys Ser Val
    1490                1495                1500

Arg Thr Tyr Asp Asn Val Thr Phe Gly Leu Asp Val Lys Thr Gly
    1505                1510                1515

Cys Glu Tyr Val Leu Thr Arg Asp Thr Ser Ser Gly Thr Pro Asp
    1520                1525                1530

Phe Thr Val Thr Phe Gln Val Val Lys Pro Asp Thr Phe Ala Lys
    1535                1540                1545

Lys Ile Arg Val Gln Leu Glu Asn Thr Leu Val Glu Leu Glu Pro
    1550                1555                1560

Phe Thr Thr Thr Asp Arg Tyr Ile Thr Val Val Val Asn Gly Thr
    1565                1570                1575

Gln Tyr Gln Ile Thr Phe Glu Lys Pro Val Val Phe Glu Tyr Ala
    1580                1585                1590

Ala Gly Lys Arg Val Phe Leu Asn Val Val Asp Thr Ser Asn Val
    1595                1600                1605

His His Ala Pro Val Ile Thr Leu Tyr Thr Glu Pro Lys Glu Val
    1610                1615                1620

Arg Val Phe Phe Asp Gly His Ser Ala Lys Val Phe Val Val Asn
    1625                1630                1635

Lys Tyr Lys Gly Asn Thr Lys Gly Val Cys Gly Asn Asn Asp Asn
    1640                1645                1650

Glu Gln Ala His Glu Phe Ile Gly Pro Asn Gly Lys Glu Tyr Gln
    1655                1660                1665

His Ala Asn Glu Phe Ile Ala Ser Tyr Gly Ile Gly Gln Ala Cys
    1670                1675                1680

Lys Val Pro Ala Glu Asn Thr Arg Glu Lys Leu Met Glu Thr Leu
    1685                1690                1695

Lys Lys Glu Val Glu Gln Ile Arg Arg Gln Glu Leu Ile Lys Lys
    1700                1705                1710

Glu Lys Leu Arg Lys Glu Met Gln Glu Leu Glu Arg Ala Arg Asn
    1715                1720                1725

Pro Gln Trp Met Glu Gln Gln Glu Glu Gln Phe Trp Gly Glu Pro
    1730                1735                1740

Leu Ser Thr Val Ser Asn Glu Glu Trp Thr Thr Asp Ser Val Glu
    1745                1750                1755

Glu Gln Gln Leu Gln Arg Gln Val Leu Lys Thr Ala Met Ser Ile
    1760                1765                1770

Glu Asn Gly His Ile Cys Phe Ser Ala Arg Pro Ile Ala Thr Cys
    1775                1780                1785

Lys Gln Gly Tyr Lys Asn His Gly Val Leu Arg Thr Glu Arg Val
    1790                1795                1800

Glu Ser Ile Cys Leu Glu Lys Asn Glu Glu Ala Ala Ile Gln Ala
    1805                1810                1815

Val Gln Glu Ile Arg Ala Gly Gln Val Val Asn Ile Lys Ser Leu
    1820                1825                1830

Glu Pro Tyr Gln Lys Gly Arg Leu Phe Thr Met His Lys Val Pro
    1835                1840                1845

Arg Cys Glu Arg His Asp Pro Glu Asn Gln Leu Ile Gln Val Trp
1850 1855 1860 1

Leu Phe Val Asn Pro Ser Thr Met Arg Phe Phe Val Leu Pro Leu Leu
5 10 15

Leu Ala Ala Ala Ala Ser Ala Glu Val Phe His Phe Val Gly Gln His
20 25 30

Gly Gln Gly Ser Thr Val Tyr Gly Val Arg Gly Ala Val Thr Val Gly
35 40 45 50

## Description

### FIELD OF THE INVENTION

The present invention relates to antigens capable of raising host immune responses to parasites. In particular, the invention provides vaccines for use in protecting against and/or reducing instances of parasite infection in avian hosts.

### BACKGROUND OF THE INVENTION

Infestation of laying-hen houses with the poultry red mite, *Dermanyssus gallinae* De Geer, costs the poultry industry an estimated €130 million per annum in the EU and infestation of production facilities with this pest has important animal welfare implications [1,2]. Poultry red mite infestation is a major health and welfare issue for laying hens as a result of anaemia, increased irritation and restlessness, feather-pecking and an increased incidence of cannibalism [1]. Poultry mites have also been implicated as intermediate hosts for a number of important diseases [3]. Controlling mite populations is currently a major problem to the egg-producing industry, with most acaricides affording only limited or short-lived reduction in the population of mites. Moreover, the withdrawal of current, effective acaricides (e.g. the organophosphate fenitrothion) along with the emergence of resistance to previously effective acaricides has exacerbated these problems with mite control [e.g. see 4]. Within Europe, varying constraints exist between countries as to the application of chemical acaricides. For example, in Sweden there are no registered compounds available for the control of ectoparasites. In a 2004 survey of UK poultry units, 87.5 % of the farms that responded reported infestation with *D. gallinae* [5].

The poultry red mite is referred to as a temporary parasite, since it is only found on the host when feeding, with the majority of its lifecycle spent concealed away in cracks and crevices of the poultry house structure. For this reason, red mite are a particular problem in free range or barn systems as opposed to caged, since a greater number of potential hiding places can be sought. In addition, EU legislation requiring the replacement of conventional cages with "enriched" cages (containing a nesting area, a roosting perch and a litter/ scratch pad) by January 2012 is likely to exacerbate the mite problem by providing more refugia for the parasites in the cages. Under optimal conditions, the lifecycle of the poultry red mite can be completed within a week, so large populations can be rapidly established. This, in conjunction with the mites' ability to occupy small spaces, makes control measures extremely difficult and there is an urgent need for alternative methods of mite control.

As an alternative control strategy, vaccination offers advantages including prolonged efficacy, freedom from chemical residues/environmental pollution and reduced risk of resistance. It is now recognised that vaccines to blood-feeding parasites can result in effective and sustainable control [6,7] including the commercial tick vaccine developed using the protective Bm86 immunogen [7]. Previous work [8,9] along with work published from other research groups [10,11] has established that vaccination against the poultry red mite, using both native and recombinant antigens, is feasible.

Previous studies have identified fractions of crude antigen preparations which raise protective responses in poultry to *D. gallinae* [8].

However, despite the wealth of research in this field a suitable vaccine against *D. gallinae* has yet to be identified. Moreover, vaccines comprising specific or single antigen candidates which are cost effective to manufacture and which offer robust protective immunity, do not exist.

The present invention seeks to obviate the problems associated with the prior art.

### SUMMARY OF THE INVENTION

The present invention is based on the finding that specific antigens derived from *Dermanyssus gallinae* (*D. gallinae*: poultry red mite) can be used to raise immune responses in avian species, prone or susceptible to, infection or infestation with/by the same. Furthermore, the inventors have discovered that the immune response raised is protective and may prevent or facilitate the clearance or eradication of a *D. gallinae* infection/infestation, in or from, an avian host. Furthermore, given the significant health problems associated with *D. gallinae* infections/infestations, the present invention may not only be used to reduce populations of *D. gallinae* infection/infestation in avian hosts, but it may also be used as a means of indirectly addressing the numerous secondary diseases and/or conditions associated with *D. gallinae* infections/infestations.

The present invention is defined by the claims appended hereto.

The invention provides one or more *Dermanyssus gallinae* (*D. gallinae*) antigens, as defined in the claims, for use in a method of raising an immune response in an avian species.

In one embodiment, the antigens for use according to this invention are provided in the form of an immunogenic composition.

The terms "avian", "avian species" or "avian host" as used herein are intended to encompass all avian species prone or susceptible to *D. gallinae* infection or infestation. In one embodiment the "avian species" encompassed by this invention include, for example, those collectively known as poultry or fowl. In other embodiments these terms extend to include domesticated or game bird species such as, for example, chicken, pheasant, grouse, turkey, guinea-fowl and/or duck species. In one embodiment, the terms "avian", "avian species" or "avian host" extend to commercially important or farmed bird species.

Advantageously, the antigens and compositions for use in methods provided by this invention may be exploited in order to raise immune responses in chickens (*Gallus gallus domesticus*). In this way, the invention provides antigens and compositions for use in methods which may be exploited to prevent, reduce and/or treat the occurrence of *D. gallinae* infections/infestations in chickens.

*D. gallinae* is a blood feeding avian ectoparasite and is exposed to host immunoglobulin when taking blood meals. Without wishing to be bound by theory, the inventors hypothesise that by administering one or more *D. gallinae* antigens to an avian, antibodies specific to (or selective for) the one or more *D. gallinae* antigens are produced in the avian's blood. In this way, when taking blood meals, *D. gallinae* parasites are exposed to the anti- *D. gallinae* antigen antibodies which adversely affect, debilitate, destroy, kill or inactivate the *D. gallinae* parasites. Again, without wishing to be bound by theory, mechanisms involved in the antibody mediated debilitation, destruction, killing and/or inactivation of the *D. gallinae* parasites may involve antibody mediated cell cytotoxicity processes and/or complement pathways.

It should be understood that while this specification makes general reference to "antibodies" and "specific" or "selective" antibodies, these terms encompass antibodies (and active or epitope binding fragments thereof) which bind to the antigens described herein (or epitopes thereof) and/or antibodies (and active or epitope binding fragments thereof) which exhibit a degree of selectivity, specificity and/or affinity therefor.

Furthermore, it should be understood that the term "antibody" may relate to polyclonal antibodies or monoclonal antibodies. Antibodies of this type are discussed later.

The description teaches vaccines or vaccine compositions comprising one or more *D. gallinae* antigens for raising immune responses in avian species. In this teaching, vaccines may be used prophylactically to prevent the establishment of a *D. gallinae* infection/infestation on an avian host or to reduce, ameliorate or eradicate an established *D. gallinae* infection/infestation. Furthermore, vaccines or vaccine compositions comprising any of the *D. gallinae* antigens described herein may be used as a means to indirectly ameliorate, reduce the symptoms of or eradicate, a secondary complication, disease or condition associated with a *D. gallinae* infection/infestation. One of skill will appreciate that the term "indirectly" ensures the reader understands that while vaccines taught herein may not have a direct effect upon secondary complications associated with *D. gallinae* infection/infestation, any reduction in the population of a *D. gallinae* infection/infestation affected by the vaccine described herein, may, in turn, affect a reduction in instances of secondary complications associated with *D. gallinae* infections/infestations. These secondary complications may be associated with pathogens (e.g.bacteria including *Salmonella, Campylobacter* or *E.coli,* mycobacterial species such as *M.gallisepticum,* or viruses including avian influenza) carried on or within the mites [4] affecting the avian host, or may be the result of the detrimental health effects on the avian host or on humans within the mites' environment, of allergens produced by the mites [12].

Without wishing to be bound by theory, it is suggested that avian species administered vaccines or vaccine compositions according to this teaching, may produce protective anti-*D*. *gallinae* antigen antibodies serving to debilitate, destroy, kill or inactivate *D. gallinae.* Additionally, and again without being bound to any particular theory, the inventors hypothesise that, depending on the specific *D. gallinae* antigen(s) exploited by this teaching, the protective antibodies raised in the avian host may have an anti-fecundity effect - restricting parasite egg production and/or larval development.

As such, in one embodiment, the present invention provides one or more *D. gallinae* antigens as defined in the claims and immunogenic compositions comprising the same for use in raising an immune response in an avian species, wherein said antigen(s) is/are (an) antigen(s) involved in a *D. gallinae* fertility process. In other embodiments, the invention provides defined antigens as specified in the claims for uses in methods (as described above) wherein the one or more *D. gallinae* antigens is/are (an) antigen(s) involved directly or indirectly in a *D*. *gallinae* fertility process. It should be understood that the phrase "antigens directly or indirectly associated with a *D. gallinae* fertility process" may encompass antigens involved in, for example, mating, fertilisation, embryogenesis, vitellogenesis, sequestration of nutrients for embryonic development, embryonic development, gender-specific reproductive and developmental processes, sexual differentiation and maturation, sexually-differentiated somatic and germ cell processes, oogenesis, spermatogenesis, oocyte maturation and/or ovulation,

The compositions, including the immunogenic compositions, provided by this invention may be formulated as sterile pharmaceutical compositions comprising one or more of the antigens described herein and a pharmaceutical excipient, carrier or diluent. These compositions may be formulated for oral, topical (including dermal and sublingual), parenteral (including subcutaneous, intradermal, intramuscular and intravenous), transdermal and/or mucosal administration.

The immunogenic compositions described herein, may comprise a discrete dosage unit and may be prepared by any of the methods well known in the art of pharmacy. Methods typically include the step of bringing into association one or more of the *D. gallinae* antigens described herein with liquid carriers or finely divided solid carriers or both.

Compositions (the term "composition" including a immunogenic compositions), suitable for oral administration wherein the carrier is a solid are most preferably presented as unit dose formulations such as boluses, capsules or tablets each containing a predetermined amount of one or more of the *D. gallinae* antigens of this invention. A tablet may be made by compression or moulding, optionally with one or more accessory ingredients. Compressed tablets may be prepared by compressing in a suitable machine an active compound (for example a *D. gallinae* antigen) in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersible granules, which may for example be suspended in water before administration, or sprinkled on food. The granules may be packaged, e.g., in a sachet. Formulations suitable for oral administration wherein the carrier is a liquid may be presented as a solution or a suspension in an aqueous or non-aqueous liquid, or as an oil-in-water liquid emulsion.

Compositions suitable for oral administration include controlled release dosage forms, e.g., tablets wherein an active compound (for example one or more *D. gallinae* antigens) is formulated in an appropriate release-controlling matrix, or is coated with a suitable release-controlling film. Such compositions may be particularly convenient for prophylactic use.

Composition and vaccine compositions formulated for parenteral administration include sterile solutions or suspensions of said defined one or more *D. gallinae* antigens in aqueous or oleaginous vehicles.

Injectable compositions and vaccines may be adapted for bolus injection or continuous infusion. Such preparations are conveniently presented in unit dose or multi-dose containers, which are sealed after introduction of the formulation until required for use. Alternatively, said one or more *D. gallinae* antigens may be in powder form that is constituted with a suitable vehicle, such as sterile, pyrogen-free water or PBS before use.

Compositions comprising one or more *D. gallinae* antigens may also be formulated as long-acting depot preparations, which may be administered by intramuscular injection or by implantation, e.g., subcutaneously or intramuscularly. Depot preparations may include, for example, suitable polymeric or hydrophobic materials, or ion-exchange resins. They may also include preparations or adjuvants known to enhance the affinity and/or longevity of the avian immune response, such as single or double emulsions of oil in water. Such long-acting compositions are particularly convenient for prophylactic use.

Compositions suitable (or formulated) for mucosal administration include compositions comprising particles for aerosol dispersion, or dispensed in drinking water. When dispensed such compositions should desirably have a particle diameter in the range 10 to 200 microns to enable retention in, for example, the nasal cavity; this may be achieved by, as appropriate, use of a powder of a suitable particle size or choice of an appropriate valve. Other suitable compositions include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

It should be understood that in addition to the carrier ingredients mentioned above, the various compositions described herein may include, an appropriate one or more additional (pharmaceutically acceptable) carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

Pharmaceutically acceptable carriers are well known to those skilled in the art and include, but are not limited to, 0.1 M and preferably 0.05 M phosphate buffer or 0.8% saline. Additionally, pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Compositions suitable for topical formulation may be provided for example as gels, creams or ointments.

One of skill will appreciate that when treating or preventing ectoparasite infections/infestations, the generation of local (cutaneous or sub-cutaneous) immune responses may be particularly advantageous.

Compositions for veterinary use may conveniently be in either powder or liquid concentrate form. In accordance with standard veterinary formulation practice, conventional water-soluble excipients, such as lactose or sucrose, may be incorporated in the powders to improve their physical properties. Thus particularly suitable powders of this invention comprise 50 to 100% w/w and preferably 60 to 80% w/w of the active ingredient(s) (for example one or more *D. gallinae* antigens) and 0 to 50% w/w and preferably 20 to 40% w/w of conventional veterinary excipients. These powders may either be added to, for example, avian feed - perhaps by way of an intermediate premix, or diluted in animal drinking water.

Liquid concentrates of this invention suitably contain one or more *D. gallinae* antigens and may optionally further include an acceptable water-miscible solvent for veterinary use, for example polyethylene glycol, propylene glycol, glycerol, glycerol formal or such a solvent mixed with up to 30% v/v of ethanol. The liquid concentrates may be administered to the drinking water of animals.

In general, a suitable dose of the one or more *D. gallinae* antigens provided by this invention may be in the range of about 10 to about 1000 µg *per* bird. Furthermore, the one or more antigens described herein may be administered on about 2 to about 5 occasions over a period of about 1 to about 10 weeks. In one embodiment, each bird may be administered about 10, 20, 30, 40, 50, 60, 70, 80, 90, 100, 150, 200, 250, 300, 350, 400, 450, 500, 550, 600, 650, 700, 750, 800, 850, 900, 950 or 1000 µg of the one or more antigens described herein. Furthermore, each bird may be administered the antigen(s) on 2, 3, 4 or 5 occasions over a 1, 2, 3, 4, 5, 6, 7, 8, 9 or 10 week period. It should be understood that each bird may receive the same or a different dose of the *D. gallinae* antigen(s) on each administration occasion.

Transdermal administration may be achieved with the use of impregnated coverings dressings, bandages or the like or via the use of some form of transdermal delivery device.

It should be understood that the term "*D. gallinae* antigen(s)" in the context of the present invention relates to a protein or protein fragment which comprises SEQ ID NO:1 or is at least 99% identical thereto.

The one or more antigens provided by this invention may be one or more antigens derived from cells or tissues involved in, or associated with, fertility (or fecundity) processes. In this way, antibodies which bind to or recognise antigens derived from cells or tissues involved in or associated with fertility or fecundity processes may inhibit or otherwise negatively affect (i) the ability of *D. gallinae* to produce viable eggs and (ii) larval development.

Antigens according to this invention may be provided as preparations derived directly from *D. gallinae.* For example, antigens for use in this invention may be obtained from whole or fragmented parasites harvested from donor animals and/or from the environment or habitat of the avian. Donor animals may be naturally infected/infested animals or animals which have been deliberately (or experimentally) infected with *D. gallinae.* In one embodiment, *D. gallinae* from which antigens may be derived may be obtained from a number of different donor avian subjects - those avian subjects inhabiting the same or different environments and/or habitats. Where the avian species are farmed, the *D. gallinae* may be obtained from one or more sites within a farm (for example a particular pen, poultry house, cage or shed within the farm) and/or from one or more of the infected or infested avian hosts within said site.

It should be understood that an immunogenic composition intended for use on a specific population of avian subjects infected/infested with *D. gallinae* (or at risk of infection/infestation with the same), may comprise one or more *D. gallinae* antigens derived from (i) one or more *D. gallinae* collected or harvested from the environment, habitat or locale of the specific population to be vaccinated and/or (ii) one or more *D. gallinae* collected or harvested from an environment, habitat or locale linked or associated with the specific population to be vaccinated. By way of example, where a farm comprises a complex of sites, perhaps spread across a particular geographical area, the invention may exploit one or more *D. gallinae* antigen(s) obtained or harvested from one or more of the sites of the complex - these antigens could be used to provide a vaccine for use on those avian subjects farmed at each site of the complex.

One of skill may refer to vaccines of the type described in the two paragraphs above as "autologous vaccines". As such, the invention teaches autologous vaccines comprising said defined one or more *D. gallinae* antigens for use in raising immune responses in avian species.

In order to obtain *D. gallinae* antigens, harvested or collected *D. gallinae* may be subjected to a homogenisation protocol to yield a homogenised suspension of *D. gallinae* components. The resulting homogenised *D. gallinae* suspension may then be subjected to one or more size/density separation techniques (such as centrifugation) so as to remove unwanted *D. gallinae* debris from useful antigen containing fractions of the suspension. The antigen containing fractions may then be subject to sterilisation procedures to render them suitable for use.

*D. gallinae* antigens for use in this invention may be further prepared for cold or freeze storage by the addition of one or more cryopreservative/cryoprotectant agents.

*D. gallinae* preparations produced in accordance with the methods described above may be regarded as "crude antigen preparations".

Crude antigen preparations of the type described above may be further processed in order to yield antigen fractions comprising fewer and more highly purified (or cleaner)/concentrated antigens or specific or select antigens. By way of example, techniques such as, for example, anion exchange, gel filtration and/or affinity chromatography may be used to prepare one or more fractions of, or to extract one or more specific antigens from, the crude antigen preparations described above.

The present description teaches that the specific *D. gallinae* antigens may be identified or characterised by PAGE analysis (i.e. migration) of *D. gallinae* antigen preparations produced in accordance with the various methods described herein and mass spectrometry analysis of protein samples subjected to PAGE analysis.

In this regard the disclosure may comprise a *D. gallinae* antigen characterised by migration in SDS (i.e. reducing) PAGE techniques as having a size/weight of approximately 80KDa and/or a *D. gallinae* protein characterised by migration in SDS (i.e. reducing) PAGE techniques as having a size/weight of approximately 120KDa.

The one or more *D. gallinae* antigens may comprise a vitellogenin protein, or pre-cursor or derivative/processed product thereof.

In accordance with the invention, the one or more *D. gallinae* antigens provided by this invention comprise a sequence which is at least 99% identical to the sequence given as SEQ ID NO: 1 below.

One of skill will appreciate that a level of sequence identity may be determined by comparing aligned amino acid sequences over a predetermined length so as to determine the number of positions at which an identical amino acid residue occurs in both sequences to yield the number of matched positions, dividing the number of matched positions by the total number of amino acid residues in the length compared and multiplying the result by 100 to yield the percentage of sequence identity.

SEQ ID NO: 1 has the following sequence:

1 VWLFVN_PST MRFFVLPLLL AAAASAEVFH FVGQHGQGST VYGVRGAVTV
51 GAHQLTAEKT ALEYNGTLAV EQIREGEFLT KFTHFTVGKY NKLQRSVQDE
101 TFDDLTPEEQ RVVKTLREPA VYEPHMQRPV RFFVKEGQIV RMEAEKEHPQ
151 WSLNIFRSVL TLFQNQVSKP ATLAVPHVEY KYEDGITGNC KVQYEVFSLP
201 EDVTVQGVFN LTKTKNYKDC LGRPVYLHLK DTQRGCAGVC DNHRPENFLA
251 GYEEEITDYE LKPTPGCPVN QQRKDTLVTV QTVTKYNVSN GYLDEVRSEH
301 TDIYRLYGGK LHVFTTLQLR LYGVAGPKIE EPKTVEIYKT LQLRLPHEED
351 ELDIPVYALL REHTTQQQYG QHFQKYFEAV VQELLQLKDT QKQGQPEKQH
401 YHSTAYLVEL VQAVSSMTEK ELKSIIPTIV HQAQPKQLTE EEHVRRQLWV
451 ELLGKAGSKS AVKIIVELVK GKLLTPTEIR RVLQDVAAFQ SYPDTEMVEQ
501 ILALCVKEQG LTATGKATAC VAAGKVLSKA CNSKVYQLAQ KHEQHKKTIN
551 GKYQSIVQMQ EQKYTPETEP EAEEYRVTFG HLPVDPKLVC TPEKLQKYVS
601 DLSHALHQAT DFKHVVAYIN GLAHVQKPEV LPELLGYVNG TASNLVHIHE
651 QGEDIKEAVE FARHVAIVSL QHVAVKYPKE VNPIVRVVFE NTTEKVQTRI
701 LAFDVWMDTQ PAQWEVEKVM QIANKDSSLE LTHYVYTALK TAMKAEEPCY
751 QLLAQRVRAA WTQLRPFDLG SEFSHLRSKF YYDTVENYGI RGVWKVIASN
801 TTILPFYTEA KVNQVRGPYK TTLFGAKLLV KGGDKVLEEL VGKDGLLERI
851 AYALVGQIKT GPRQQNTEQL LKDIAQGMGL KREKDETPKA VLFWKLFSGD
901 AVIPLDSHYI NELKQELLQT VTKFGKDGVT GHIVRVLVPT KAFHVEPSTI
951 GLPIVHSTIH PVVLSVRYEN IKIHYGNQES RVAPKTLEIS GTVQPTILSF
1001 RQSRVFVSDK VGQKNPTVKT TDIKEFNVRL AFRVVYEHTP KRFRVHVKPV
1051 FDRVFHSGHC TELKLESAVL LKEELAAKTV EYDKCIKSLY QPIRRNHQIA
1101 GEWSGMMLRL TGESHQPWSG LPMFAPSVVS SEGILGAIIN RLSNKGMKHH
1151 TVSLYLETNN QQPITEWVAT IDVDSNVERL AKVPLSQQIT KVQKLKVQYA
1201 NRAQPLYPEL EPLVRKVESL LEKFETLDET TVEKLMLVKI EGLYQGQPKS
1251 TLKIAMKKIY NLEKTEQQYA LAAMHQESHK GLELSTNVSY PKIGSPFRYD
1301 PTFYAEDERM NGTLIVKLQS PQEQVFHVKF QATKSEEQLK ETEYEWFEVR
1351 CLAEQKAGKI MTDACRKAVL KDNSLDQLKI AVTVPRNVHP KIQTLAYKTL
1401 DLMKYMWYPK MQTEVAGLKQ REVLQALQHT EREVRISVNA TRESLWHLLY
1451 DVRVEMPFEN VTFSKVNIPG VRPAHMQLTT KEQLEHVYYR GQKDNVCVLG
1501 DKSVRTYDNV TFGLDVKTGC EYVLTRDTSS GTPDFTVTFQ VVKPDTFAKK
1551 IRVQLENTLV ELEPFTTTDR YITVVVNGTQ YQITFEKPVV FEYAAGKRVF
1601 LNVVDTSNVH HAPVITLYTE PKEVRVFFDG HSAKVFVVNK YKGNTKGVCG
1651 NNDNEQAHEF IGPNGKEYQH ANEFIASYGI GQACKVPAEN TREKLMETLK
1701 KEVEQIRRQE LIKKEKLRKE MQELERARNP QWMEQQEEQF WGEPLSTVSN
1751 EEWTTDSVEE QQLQRQVLKT AMSIENGHIC FSARPIATCK QGYKNHGVLR
1801 TERVESICLE KNEEAAIQAV QEIRAGQVVN IKSLEPYQKG RLFTMHKVPR
1851 CERHD_PE_N Q_LIQ

In one embodiment, the antigens and compositions for use in methods provided by this invention comprise one or more *D. gallinae* antigens comprising a sequence selected from the group consisting of:
(a) SEQ ID NO: 1, or
   a sequence at least 99% identical to (a). In one embodiment, the invention provides an immunogenic composition comprising one or more *D. gallinae* antigens comprising a sequence selected from the group consisting of:
   (a) SEQ ID NO: 1, or
   (b) a sequence at least 99% identical to (a).
wherein said composition is for use in raising an immune response in an avian. In one embodiment, the avian species is a chicken (*Gallus gallus domesticus*). In a further embodiment, the immune response protects against *D. gallinae* infection/infestation.

In addition to providing specific peptide sequences, the invention further provides nucleic acid molecules encoding the same. The nucleic acid may be DNA, RNA or a combination thereof and can include any combination of naturally occurring, chemically or enzymatically modified nucleotides. Furthermore, the nucleic acid may be double or single stranded. Within the scope of this invention are nucleic acid sequences that are substantially complementary to any nucleic acid sequence encoding SEQ ID NO: 1 described herein.

It should be understood that the term "substantially complementary" encompasses those nucleic acid molecules exhibiting a degree of sequence identity with any nucleic acid sequence encoding SEQ ID NO: 1 above. A nucleic acid sequence having a level of identity with a sequence encoding SEQ ID NO: 1 exhibits at least 99% identity with the full length nucleic acid sequence encoding SEQ ID NO: 1.

One of skill will appreciate that a level of sequence identity may be determined by comparing aligned nucleic acid sequences over a predetermined length so as to determine the number of positions at which an identical nucleic acid base occurs in both sequences to yield the number of matched positions, dividing the number of matched positions by the total number of nucleic acid bases in the length compared and multiplying the result by 100 to yield the percentage of sequence identity.

In view of the above, the present invention relates to antigens (and compositions comprising the same), use in methods exploiting (i) proteins encoded by the sequences designated SEQ ID NO: 1 above as well as proteins encoded by nucleic acid sequences encoding any of SEQ ID NO: 1. Furthermore, the invention relates to antigens (and compositions), use in methods exploiting any variants of SEQ ID NO;1 which is at least 99% identical thereto.

The disclosure may further relate to natural or artificially created variants or analogues of any of the protein/nucleic acid sequences described herein. Such variants may exhibit one or more amino acid/nucleic acid deletions, additions, substitutions and/or inversions, relative to SEQ ID NO: 1. In certain embodiments, the substitutions may represent conservative substitutions. One of skill will appreciate that a conservative substitution involves replacing one or more amino acids of a protein or peptide sequence with an alternate amino acid having similar properties and which does not substantially alter the physio-chemical properties and/or structure or function of the native (or wild type) protein.

As is well known in the art, the degeneracy of the genetic code permits substitution of one or more bases in a codon without alteration to the primary amino acid sequence. As such, genetic degeneracy may be exploited in order to yield variant nucleic acid sequences which encode peptide or protein sequences of SEQ ID NO; 1.

The nucleic acid molecules provided by this invention may take the form of nucleic acid constructs or vectors such as, for example a cloning or expression cassettes/vectors including phage vectors for use as described, for example in EP1370284. The term "nucleic acid constructs" or "vector" may further encompass constructs intended for use as DNA vaccines.

Vectors provided by this invention may be capable of directing the expression of nucleic acid sequences encoding *D. gallinae* antigens in, for example, bacterial, fungal, animal (including avian species and mammalian) and/or insect cells.

Expression vectors suitable for use in the invention may further comprise one or more promoter sequences capable of directing expression in prokaryotic or eukaryotic cells such as, for example, avian, mammalian, fungal, bacterial, plant and/or insect cells.

A vector provided by this invention may be circular or linear, single stranded or double stranded and can include DNA, RNA or a combination or modification thereof. Furthermore, vectors of this invention may be, for example, plasmids, cosmids or viral vectors (for example retroviral or bacteriophage vectors). Vectors provided by this invention may further comprise selection or marker elements, for example antibiotic resistance genes and/or optically detectable tags. A large number of suitable vectors are known and further information may be obtained from Pouwels et al. Cloning Vectors: a Laboratory Manual (1985 and supplements), Elsevier, N.Y.; and Rodriquez, et al. (ads.) Vectors: a Survey of Molecular Cloning Vectors and their Uses, Buttersworth, Boston, Mass (1988) - both of which are incorporated herein by reference.

As such, in addition to techniques in which *D. gallinae* antigens are extracted or purified directly from harvested or collected *D. gallinae,* antigens to be exploited in this invention may be obtained using recombinant technology. In one embodiment, an expression vector comprising one or more nucleic acid sequences encoding a *D. gallinae* antigen as defined herein may be used to produce one or more recombinant *D. gallinae* antigens.

The present disclosure teaches host cells transfected or transformed with a vector as described herein. Eukaryotic or prokaryotic cells, such as, for example avian, plant, insect, mammalian, fungal and/or bacterial cells, may be transfected with one or more of the vectors described herein. One of skill in this field will be familiar with the techniques used to introduce heterologous or foreign nucleic acid sequences, such as expression vectors, into cells and these may include, for example, heat-shock treatment, use of one or more chemicals (such as calcium phosphate) to induce transformation/transfection, the use of viral carriers, microinjection and/or techniques such as electroporation. Further information regarding transformation/transfection techniques may be found in Current Protocols in Molecular Biology, Ausuble, F.M., ea., John Wiley & Sons, N.Y. (1989) which is incorporated herein by reference.

The present disclosure further teaches a process for the production of a *D. gallinae* antigen encoded by a sequence of SEQ ID NO: 1 and for use in raising an immune response in an avian, said method comprising the step of (a) transforming a host cell with a nucleic acid sequence according to this invention or transfecting a host cell with a nucleic acid construct of the invention; (b) culturing the cells obtained in (a) under conditions in which expression of the nucleic acid (or rather a protein encoded thereby) takes place; and (c) isolating the expressed recombinant protein or peptide from the cell culture and/or the culture supernatant.

Recombinant proteins/peptides produced according to the method described above may be partially purified from the host cell before being used in a vaccine or vaccine composition. Where the polypeptide is secreted from the host cell, the cells may be separated from the media by centrifugation. In such a situation, the supernatant, which contains the secreted polypeptide, may be used directly as a vaccine, or in a vaccine composition. Alternatively, the polypeptide may be partially purified from this supernatant, for example using affinity chromatography.

In one embodiment, any of the *D. gallinae* antigens described herein (whether directly isolated from harvested or collected *D. gallinae* or recombinantly produced) may be admixed with another component, such as another polypeptide and/or an adjuvant, diluent or excipient. Vaccines or vaccine compositions provided by this invention may, for example, contain viral, fungal, bacterial or other parasite antigens used to control other avian diseases/infections or infestations. For example, the vaccine or vaccine composition may be included within a multivalent vaccine which includes antigens against other avian (for example chicken) diseases. These vaccines may include, for example, the vaccines and target organisms listed in Table 1, below.

**Table 1. Example avian vaccines.**

| **Vaccine name** | **Manufacturer** | **Target organism** |
|---|---|---|
| 89/03 | MSD AH [Intervet] | Avian Infectious bursal disease virus [IBDV, Gumboro disease virus] |
| ABRONCHOVAX | Laboratoire National Vétérinaire [LANAVET] | Avian infectious bronchitis virus [IBV] |
| ADENIPRAVAC ND/IB | Laboratorios HIPRA S.A. | Avian infectious bronchitis virus [IBV], Egg drop syndrome virus [EDS virus, Avian adenovirus], Newcastle disease virus [NDV, Avian paramyxovirus, APMV-1] |
| ADVENT Coccidiosis Control | Viridus Animal Health L.L.C. [Novus International Inc.] | Eimeria acervulina, Eimeria tenella, Eimeria maxima |
| AE+Pox | MSD AH [Intervet] | Avian encephalomyelitis virus [AEV], Fowl pox virus |
| AE-POX VACCINE NOBILIS | MSD AH [Intervet] | Fowl pox virus, Hepatovirus |
| Aerovac Al | Investigación Aplicada, S.A. de C.V. | Avian influenza virus [Al] |
| Anemovac | Investigación Aplicada, S.A. de C.V. | Chicken infectious anaemia virus [CIAV] |
| Angara Disease | Sindh Poultry | Adenovirus |
| Vaccine | Vaccine Centre (SPVC) | |
| ART VAX | MSD AH [Intervet] | Bordetella avium (Attenuated) |
| Artri-Vet | Laboratório Bio-vet S.A. | Avian reovirus (S 1133) |
| Avian Cholera Vaccine | Veterinary Serum and Vaccine Research Institute | Pasteurella multocida |
| Aviffa RTI | Merial | Avian rhinotracheitis virus [Avian metapneumovirus] (VCO3) (Attenuated) |
| AviPro 101 Coryza | Lohmann Animal Health GmbH & Co. KG | Haemophilus [Avibacterium] paragallinarum [Infectious coryza virus] |
| AviPro 104 MG | Lohmann Animal Health GmbH & Co. KG | Mycoplasma gallisepticum (S 6) |
| AviPro 109 SE4 | Lohmann Animal Health GmbH & Co. KG | Salmonella Enteritidis (8, 14B, 23, 24) |
| AviPro ILT | Lohmann Animal Health GmbH & Co. KG | Gallid herpesvirus 1 [GaHV-1, Avian herpesvirus 1, Infectious Laryngotracheitis virus, ILTV] |
| AviPro MD BIVAC | Lohmann Animal Health GmbH & Co. KG | Marek's disease virus |
| AviPro SALMONELLA | Lohmann Animal | Salmonella Enteritidis (Sm24/Rif12/Ssq) (Attenuated), |
| DUO | Health GmbH & Co. KG | Salmonella Typhimurium (Nal2/Rif9/Rtt) (Attenuated) |
| AviPro THYMOVAC | Lohmann Animal Health GmbH & Co. KG | Chicken infectious anaemia virus [CIAV] (CUX-1) |
| BAK-MG | Bestar Laboratories Pte Ltd. | Mycoplasma |
| BDK-PA | Bestar Laboratories Pte Ltd. | Pasteurella anatipestifer |
| Bio Coccivet R | Laboratório Bio-vet S.A. | Eimeria acervulina , Eimeria brunetti, Eimeria maxima , Eimeria necatrix , Eimeria praecox , Eimeria tenella , Eimeria mitis |
| Bio Mark Vet C | Laboratório Bio-vet S.A. | Meleagrid herpesvirus 1 [MeHV-1, Marek's disease virus serotype 3, Turkey herpesvirus, HVT] (FC126) |
| Bio-SHS | Laboratório Bio-vet S.A. | Avian metapneumovirus [Avian/Turkey rhinotracheitis] |
| Bio-SHS Viva | Laboratório Bio-vet S.A. | Avian pneumovirus |
| CALAVAC REO | C.A. Laboratorios Asociados [CALA] | Reovirus (S1133) |
| CAV Vaccine | Intervet Australia Pty Ltd. | Chicken infectious anaemia virus [CIAV] (3711) |
| Chevipok | Chevita GmbH | Pigeon pox virus |
| Chevivac-P12 | Ceva Animal Health Ltd. | Pigeon paramyxovirus 1 [PPMV-1] |
| Chicken Necrotic Enteritis Gel Vaccine | Veterinary Serum and Vaccine Research Institute | Clostridium [welchii] perfringens |
| Coli-Ave Oleosa | Laboratório Bio-vet S.A. | Escherichia coli (O1, O2, O35, O78) (Subunit) |
| COR-2 | Merial | Coronavirus (PL84084, CR88121) |
| Deparmune | Ceva Santé Animale | Barbarie duck parvovirus (FM), Derzsy disease virus [Goose parvovirus] (LB) |
| Difeterviruela Aviaria Inmuner | Laboratorios Inmuner S.A.I.Y.C. | Avian smallpox virus (Attenuated) |
| Dindoral SPF | Merial | Epizootic haemorrhagic disease [EHD] virus |
| Duck Hepatitis Virus | Green Cross | Duck hepatitis virus (DHV-HSB type I) |
| Live Freezing Vaccine | Veterinary Products Co. Ltd. | |
| Duck Virus Enteritis Vaccine | International Duck Research Cooperative Inc. | Anatid herpesvirus 1 [AHV-1, Duck herpesvirus 1] |
| Fowl Pox Vaccine Living | Ventri Biologicals [Venkateshwara Hatcheries Private Ltd.] | Fowl pox virus |
| FOWL POX VACCINE. LIVE. I. P. (Vet.) | BiO-MED Private Ltd. | Fowl pox virus (BM) (Attenuated) |
| Fowl Spirochaetosis Vaccine Inactivated | Ventri Biologicals [Venkateshwara Hatcheries Private Ltd.] | Borrelia anserina |
| Fowl typhoid | National Veterinary Institute Ethiopia | Salmonella Gallinarum (Attenuated) |
| HIPRAVIAR TRT | Laboratorios HIPRA S.A. | Turkey rhinotracheitis virus [Avian metapneumovirus] |
| Immucox Turkey | Imuvet Comercial Ltda. | Eimeria adenoeides , Eimeria meleagrimitis |
| Nobilis Erysipelas | MSD AH [Intervet] | Erysipelothrix rhusiopathiae (M2) |
| Nobilis OR Inac | MSD AH [Intervet] | Ornithobacterium rhinotracheale [ORT] ( |
| Emulsion for Injection for Chickens | | serotype A strain B3263/91) |
| Parduvak | IDT Biologika GmbH | Parvovirus |
| Parvokan | Merial | Derzsy disease virus [Goose parvovirus] (H) (Attenuated), Muscovy duck parvovirus (GM) |
| Pro'tect HE | Brinton Laboratories Inc. | Hemorrhagic enteritis virus |
| Virsin 424 | Biovac Company Ltd. | Riemerella anatipestifer , Pasteurella multocida |

The present disclosure teaches an avian population, for example a farmed population of chickens, treated, vaccinated or immunised with a vaccine or composition described herein, said vaccine or composition comprising one or more of the *D. gallinae* antigens described herein.

One of skill will appreciate that the vaccines described in this invention may take the form of subunit-type vaccines where by one or more *D. gallinae* antigens are used to inoculate an animal. Additionally or alternatively, the vaccine may comprise a nucleic acid molecule (known as a DNA vaccine) encoding one or more antigens encoded by SEQ ID NO: 1, to be expressed by the cells of an animal to be vaccinated. In this way, constitutive expression of *D. gallinae* antigens in a vaccinated host (such as, for example a vaccinated chicken) may elicit a constitutive protective immune response.

In addition to providing *D. gallinae* antigens for use in raising immune responses in animals, the present disclosure also teaches polyclonal and/or monoclonal antibodies (or antigen binding fragments thereof) that bind (or have affinity or specificity for) any of the *D. gallinae* antigens described. Production and isolation of polyclonal/monoclonal antibodies specific for protein/peptide sequences is routine in the art, and further information can be found in, for example "Basic methods in Antibody production and characterisation" Howard & Bethell, 2000, Taylor & Francis Ltd. Such antibodies may be used in diagnostic procedures, to, for example detect or diagnose *D. gallinae* infection/infestations in avian species, as well as for passive immunisation.

The present disclosure further teaches a vaccine for use in preventing or controlling *D. gallinae* infection/infestation and associated diseases in avian hosts. The vaccine may be a polypeptide or polynucleotide vaccine.

The disclosure further teaches a method for immunising an avian against *D. gallinae* infection/infestation and associated disease (for example secondary infections etc.), said method comprising the step of administering a vaccine of the invention to the avian.

### DETAILED DESCRIPTION

The present teaching will now be described in detail with reference to the following Figures which show:
Figure 1: SDS-PAGE to show protein profile of extracted antigens. Lane 1, 1/10 dilution of antigen, Lane 2, 1/100 dilution, Lane 3 Seablue plus 2 molecular mass marker.
Figure 2: Immunoblots of red mite vaccine preparation: panel A) Control - IgY taken from eggs that were not exposed to vaccination and panel B) IgY from eggs from hens vaccinated with mite antigens from a previous trial. Both panels: left to right lanes consist of molecular mass marker, then pre and post filtered antigen, respectively. Following filtration, no differences in antigenic profile were observed.
Figure 3: An Overview of the cages. Line 4 and 3 are back to back as is line 2 and line 1. Group 2 is the vaccinated group and Group 1 the control group. The gaps between group 2 and group 1 are empty cages that were treated to decrease mite migration between cages.
Figure 4: Mite numbers (/100) over a 4 month period. The control group (indicated by the blue line) are the mites that fed on hens that had been injected with adjuvant only. The vaccinated group (indicated by the red line) are the mites that fed on hens that had been injected with the vaccine antigen prepared in the same adjuvant.
Figure 5: A Western blot carried out on the antigen prepared using anti IgY extracted from the eggs collected from the vaccinated chickens and control chickens.
Figure 6: A Western blot carried out on antigen prepared using anti IgY extracted from the eggs collected from the chickens vaccinated with Formalin-inactivated antigen (Form), Beta propiolactone-inactivated antigen (BP) and control chickens (immunized with adjuvant only).

### Methods

### i) Antigen and vaccine preparation:

5mL of ice-cold phosphate buffered saline (PBS) was added to *ca.* 1mL of mites and homogenised on ice for 30sec. The homogenised mite sample was rested for 1 min on ice before repeating a second round of homogenisation for 30sec. The homogenised sample was then centrifuged for 20min @ 24000 xg @ 4°C. The supernatant (antigen) was filtered through a 0.22µm filter and divided into 1mL aliquots and stored at -80°C

Protein content of the sample was determined using the Pierce BCA protein assay, with bovine serum albumin (BSA) standards.

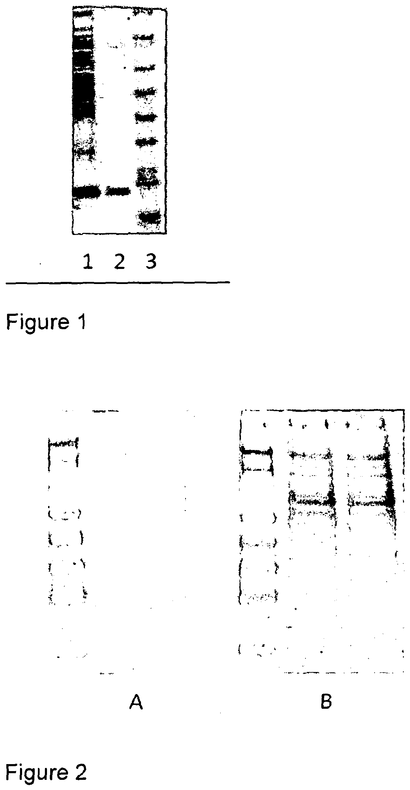

The protein profile of the extract was investigated by separation of the native extract on NuPAGE® Bis-Tris 4-12% gels, under reducing conditions, employing NuPAGE® MES SDS running buffer (Invitrogen) and the profile is shown in Figure 1.

An immunoblot was also performed on the mite extract, to determine the antibody reactivity of these antigens. The antibodies used for the blot were obtained from the eggs of hens previously vaccinated with red mite antigens, from a prior study. In addition, the impact of sterile filtration (to remove potentially harmful bacteria and sterilize the vaccine) on the antigen profile was also investigated, by comparing the immunoreactive antibody profiles before and after filtration (see Figure 2).

On the basis of the integrity of the proteins and their immunoreactivity, we proceeded to generate the vaccine. A total of 285 mgs (post filtration) of red mite protein was couriered on dry ice to Ridgeway Biological Ltd, Compton, for formulation in an oil-in-water emulsion. Two equal batches of 250mls of the vaccine were produced (total 500 ml containing 285micrograms per 0.5ml dose), and, after confirmation of sterility, these two batches were couriered directly to Roslin Nutrition Ltd.

### ii) Experimental plan and cage layout:

The trial involved a total of 768 Lohmann Brown hens, raised on-site at Roslin Nutrition Ltd. with no prior exposure to *D. gallinae,* consisting of two groups of hens. One group was assigned as the vaccine group (receiving the vaccine), and the second represented normal (receiving adjuvant alone) control hens. Neither group was treated with chemicals employed to control red mites during the duration of the study. All birds received two intra muscular injections at 12 and 17 weeks of age, before being assigned to cages. The vaccinated birds received 0.5ml of vaccine containing 285 micrograms of mite soluble protein/injection, with controls receiving an equal volume of adjuvant alone. After the second vaccine injection, the hens were placed in cages (4 per cage) in an isolated housing unit. This unit was free of red mites before the commencement of the study. Cages were placed together to form blocks consisting of four by three cages, and arranged to form two rows of blocks separated by a 1 metre passage. Each block was interspaced by empty cages lined with 'mite proof' plastic sheeting. Blocks consisted of either vaccinated or control hens, in an alternated pattern to reduce any 'space effect' within the housing unit between the two groups.

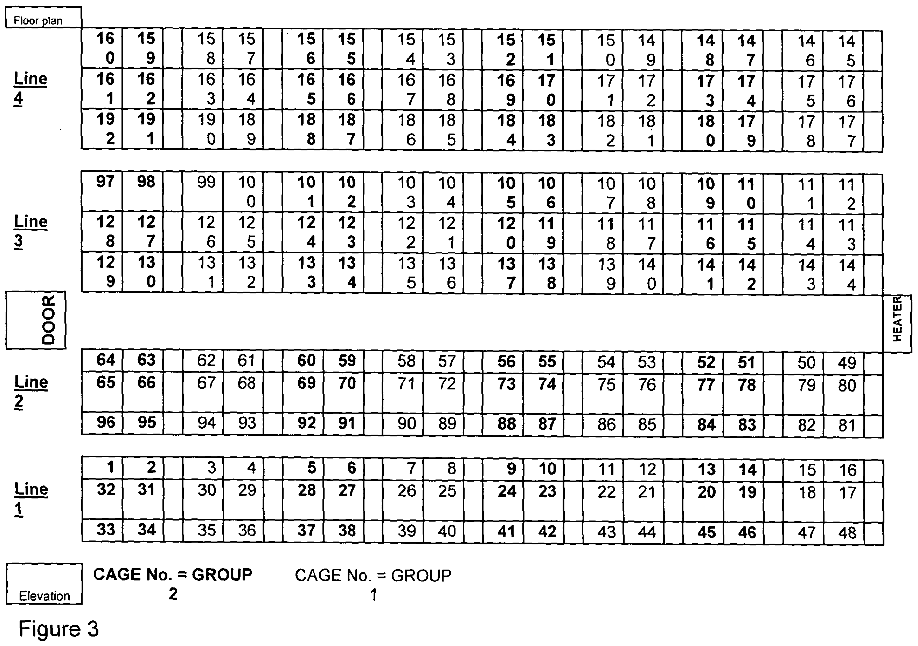

A diagram depicting the cage layout is shown in Figure 3.

The groups were designated by Roslin Nutrition as 'Group 1' and 'Group 2', and information on what treatment was given to each group was withheld from the scientists until after the mite counts were completed, to allow a "blind" trial.

### Mite counts:

Throughout the period of study, red mite numbers were assessed by standard trapping and counting techniques. ADAS traps were placed into each cage 24 hours before collection, and introduced 2 weeks after the mite challenge. On collection traps were treated with 70% ethanol to kill the mites and placed in sealed containers. Mites were counted in a 'blind' fashion (i.e. without the counter's knowledge of which group was which), being designated with cage number only. On collection, mites were washed from the traps and containers with 70% ethanol into Petri dishes, before being counted. The mites were usually large enough to be visualized by the naked eye, and a dissecting microscope was only employed where there was doubt between distinguishing between debris and mite. Initial counts were very low (see results), but in the latter half of the study mite numbers increased substantially. In order to count these high numbers (over 500 mites/trap), the Petri dish was divided into equal portions by placing onto graticuled paper, the contents of a limited number of the portions were counted and the total number estimated by multiplication from these counts.

### Production and welfare:

Egg production and general health parameters/observations of both groups were assessed on a regular basis throughout the study.

### Further development of the vaccine towards industry compliance

The practical use of any autogenous vaccine will require Veterinary Medicine Directorate (VMD) approval. To obtain this approval we need to ensure freedom from adventitious agents. The most straightforward method of doing this is by "inactivation" of the vaccine by treatment of the mite extract with formalin or beta-propiolactone. Thus, the extract, prepared as described above ("Antigen and vaccine preparation") was treated with either formalin or beta-propiolactone prior to formulation with a standard autogenous vaccine preparation (containing inactivated *Escherichia coli, Pasteurella multocida, Mycoplasma gallisepticum* and *M. synoviae* antigens) by a commercial autogenous vaccine producing company, and injected into laying hens (in duplicate). Antibodies were extracted from the eggs laid by these hens and their immunoreactive profile was examined by immunoblot to establish whether comparable antigenicity was achieved between these inactivated extracts and the original extracts which had been used in the field trial described above. Ultimately, these antibodies will be mixed with hen blood and fed to red mites *via* an *in vitro* feeding device for their anti-mite effect, by determining mortality over the following 5 day period.

### Results

### Antigen preparation and vaccination:

No problems were encountered during antigen preparation or formulation for the vaccine.

### Mite counts:

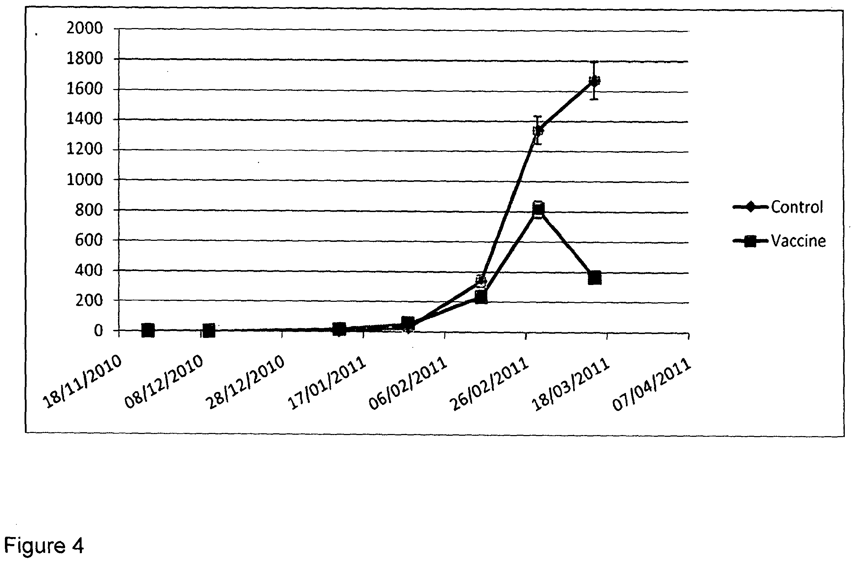

Mite numbers were counted and recorded for each sampling date (see Table 1). A graph was then generated using the values that were recorded for each date (see Figure 4)

**Table 1: The sum of the different sets of cages (6 cages in one set)**

| | **25/11/2010** | | **10/12/2010** | | **11/01/2011** | | **28/01/2011** | | **15/02/2011** | | **01/03/2011** | | **15/03/2011** | |
|---|---|---|---|---|---|---|---|---|---|---|---|---|---|---|
| | **Grp 2** | **Grp 1** | **Grp 2** | **Grp 1** | **Grp 2** | **Grp 1** | **Grp 2** | **Grp 1** | **Grp 2** | **Grp 1** | **Grp 2** | **Grp 1** | **Grp 2** | **Grp1** |
| set 1 | 2 | 6 | 0 | 0 | 19 | 170 | 53 | 495 | 2685 | 7713 | 8899 | 17134 | 4029 | 32377 |
| set 2 | 18 | 2 | 0 | 0 | 237 | 5 | 961 | 280 | 3778 | 2616 | 9275 | 13157 | 5852 | 12940 |
| set 3 | 4 | 8 | 0 | 1 | 2 | 28 | 141 | 706 | 2268 | 5947 | 11979 | 23809 | 3821 | 39439 |
| set 4 | 14 | 12 | 1 | 1 | 573 | 309 | 1211 | 368 | 3468 | 4670 | 15081 | 15350 | 6090 | 14751 |
| set 5 | 1 | 15 | 1 | 0 | 29 | 50 | 480 | 853 | 1391 | 4738 | 7247 | 18952 | 2448 | 17627 |
| set 6 | 26 | 11 | 0 | 0 | 386 | 25 | 1774 | 168 | 2988 | 1537 | 9080 | 10580 | 6133 | 14331 |
| set 7 | 14 | 1 2 | 1 | 0 | 45 | 14 | 235 | 103 | 2040 | 2974 | 5669 | 19636 | 3736 | 21417 |
| set 8 | 14 | 5 | 1 | 4 | 339 | 18 | 547 | 86 | 3292 | 2722 | 9936 | 8880 | 3052 | 10767 |
| | | | | | | | | | | | | | | |
| **Sum** | **93** | **71** | **4** | **6** | **1630** | **619** | **5402** | **3059** | **21910** | **32917** | **77166** | **127498** | **35161** | **163649** |

From these results, it can be seen that for the first few weeks after mite challenge, very few mites were recorded in the traps. However, at the beginning of February and approximately 9 weeks following the mite challenge, the mite counts increased and by the end of the study, substantial numbers were recorded. At this time, highly significant differences (P>0.01 and P>0.001 at time points 01/03/2011 and 15/03/2011, respectively) in these counts between the vaccinated and control groups were demonstrated, with mean numbers in the vaccinated group approximately four-fold less than those in the control group. Of particular note during these counts was the observation that there were markedly fewer nymphal (juvenile) mites in traps from the vaccinated group when compared to those in the control group, where they constituted a sizeable proportion (∼50%) of the total number. Further quantitative studies are required to confirm these observations.

### Immunological studies:

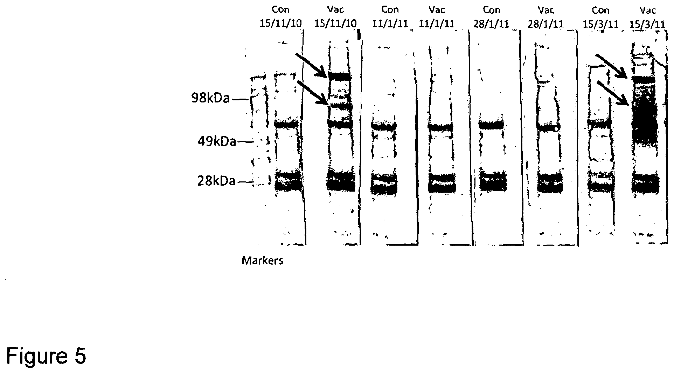

The antigen prepared for the vaccination was separated by electrophoresis and used in an immunoblot to determine the antigens to which the highest antibody response was mounted during the trial. Eggs were collected 2 weeks post vaccination (15/11/2010), one month post vaccination (11/01/2011), 2 month post vaccination (28/01/2011) and the last collection at the end of the study (15/03/2011). Immunoglobulin prepared from these eggs was used to probe the immunoblots of the vaccine antigens. These blots are shown in figure 5. Although strong antibody reactive bands are seen from both the control and vaccinated antibodies, those from the former are likely to be avian IgY (heavy and light chains) extracted from the mites which contained ingested chicken blood, detected with the secondary anti-IgY antibody. Following vaccination with mite antigens however, additional antigenic bands were observed, with two of the most prominent being in the order of ∼80KDa and 120KDa (arrowed in Fig 5). It is relevant that antibodies to both these proteins appear to decline after vaccination, but reappear and are strongly represented at the terminal stages of the study when there was a significant divergence in mite populations between the two groups.

The immunoreactive (reacting with chicken IgY derived from hens vaccinated with a PBS extract of *Dermanyssus gallinae*) bands on Western blots of PBS extract of *Dermanyssus gallinae* were aligned with the corresponding area on an SDS PAGE gel (1 dimensional) separation of the same extract. The bands were excised and analysed by MALDI-Tof to derive a peptide mass fingerprint which was used to interrogate an EST database of nucleic acid sequences derived from *D. gallinae* mRNA. The EST database was produced by and held at Moredun Research Institute. It is not publically available. The contigs with the highest scores (sequence coverage) for each of the immunoreactive bands were translated into amino acid sequence and used to interrogate the NCBI BLASTp (nr) public database to assign identity and function. Each of the bands possessed very high homology to invertebrate vitellogenins (See Example 1: E< 1 x10⁻⁸⁰).

### Further development of the vaccine towards industry compliance

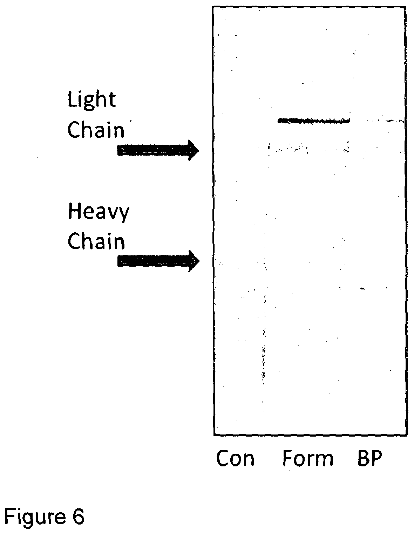

Antibodies were extracted from the eggs laid by hens which had been immunized with red mite extract, prepared as described above ("Antigen and vaccine preparation") and treated with either formalin or beta-propiolactone prior to formulation with a standard autogenous vaccine preparation (containing inactivated *Escherichia coli, Pasteurella multocida, Mycoplasma gallisepticum* and *M. synoviae* antigens) by Ridgeway Biological Ltd., Compton, Berkshire. The immunoreactive profile was examined by immunoblot to establish whether comparable antigenicity was achieved between these inactivated extracts and the original extracts which had been used in the field trial described above. Following vaccination with inactivated mite antigens (using either formalin or beta propiolactone) antigenic bands were observed, with two of the most prominent being in the order of ∼80KDa and 120KDa (Figure 6), and similar to the western blot profile observed with antibodies obtained from the vaccinated hens in the *in vivo* trial described above.

### Conclusions

This trial has provided strong evidence that vaccination of hens with an autogenous vaccine prepared from poultry red mites can have a major effect on mite populations, and provide alternative control for this parasite. Inactivation of the antigens using formalin or beta propiolactone did not affect their immunogenicity, indicating the commercial viability and exploitation for an autogenous vaccine for the poultry red mite.

The results strongly suggest that antibodies generated following vaccination affected mite survival and/or mite fecundity. That the latter may be the case is suggested by our observation that fewer juvenile mites were present in the vaccinated group; such an observation is in accord with an 'anti-fecundity' effect influencing egg production or larval development.
This field trial has provided proof-of-concept data to support the use of as a vaccine to control poultry red mite.

### Example 1

Three immunoreactive (reacting with chicken IgY derived from hens vaccinated with a PBS extract of *Dermanyssus gallinae*) bands on Western blots of PBS extract of *Dermanyssus gallinae* were aligned with the corresponding area on an SDS PAGE gel (1 dimensional) separation of the same extract. The bands were excised and analysed by MALDI-Tof to derive a peptide mass fingerprint which was used to interrogate an EST database of nucleic acid sequences derived from *D. gallinae* mRNA. The EST database was produced by and held at Moredun Research Institute. It is not publically available. The contigs with the highest scores (sequence coverage) for each of the immunoreactive bands were translated into amino acid sequence and used to interrogate the NCBI BLASTp (nr) public database to assign identity and function. The three proteins for identification are labeled JH 1-3:
>JH 1
Match to: **contig12013** Score: **344** Expect: **3.2e-30**
**length=5596 numreads=1668**
Translated in frame 5
Nominal mass (Mᵣ) : **212498;** Calculated pI value: **8.06**
NCBI BLAST search of contig12013 against nr
Unformatted sequence string for pasting into other applications
Variable modifications: Carbamidomethyl (C),Oxidation (M),Propionamide (C) Cleavage by Trypsin: cuts C-term side of KR unless next residue is P
Number of mass values searched: **81**
Number of mass values matched: **58**
Sequence Coverage: **33%**
Matched peptides shown in **Bold** 
1 VWLFVN_PST MRFFVLPLLL AAAASAEVFH FVGQHGQGST VYGVRGAVTV
51 GAHQLTAEKT ALEYNGTLAV EQIREGEFLT KFTHFTVGKY NKLQRSVQDE
101 TFDDLTPEEQ RVVKTLREPA VYEPHMQRPV RFFVKEGQIV RMEAEKEHPQ
151 WSLNIFRSVL TLFQNQVSKP ATLAVPHVEY KYEDGITGNC KVQYEVFSLP
201 EDVTVQGVFN LTKTKNYKDC LGRPVYLHLK DTQRGCAGVC DNHRPENFLA
251 GYEEEITDYE LKPTPGCPVN QQRKDTLVTV QTVTKYNVSN GYLDEVRSEH
301 TDIYRLYGGK LHVFTTLQLR LYGVAGPKIE EPKTVEIYKT LQLRLPHEED
351 ELDIPVYALL REHTTQQQYG QHFQKYFEAV VQELLQLKDT QKQGQPEKQH
401 YHSTAYLVEL VQAVSSMTEK ELKSIIPTIV HQAQPKQLTE EEHVRRQLWV
451 ELLGKAGSKS AVKIIVELVK GKLLTPTEIR RVLQDVAAFQ SYPDTEMVEQ
501 ILALCVKEQG LTATGKATAC VAAGKVLSKA CNSKVYQLAQ KHEQHKKTIN
 
551 GKYQSIVQMQ EQKYTPETEP EAEEYRVTFG HLPVDPKLVC TPEKLQKYVS
601 DLSHALHQAT DFKHVVAYIN GLAHVQKPEV LPELLGYVNG TASNLVHIHE
651 QGEDIKEAVE FARHVAIVSL QHVAVKYPKE VNPIVRVVFE NTTEKVQTRI
701 LAFDVWMDTQ PAQWEVEKVM QIANKDSSLE LTHYVYTALK TAMKAEEPCY
751 QLLAQRVRAA WTQLRPFDLG SEFSHLRSKF YYDTVENYGI RGVWKVIASN
801 TTILPFYTEA KVNQVRGPYK TTLFGAKLLV KGGDKVLEEL VGKDGLLERI
851 AYALVGQIKT GPRQQNTEQL LKDIAQGMGL KREKDETPKA VLFWKLFSGD
901 AVIPLDSHYI NELKQELLQT VTKFGKDGVT GHIVRVLVPT KAFHVEPSTI
951 GLPIVHSTIH PVVLSVRYEN IKIHYGNQES RVAPKTLEIS GTVQPTILSF
1001 RQSRVFVSDK VGQKNPTVKT TDIKEFNVRL AFRVVYEHTP KRFRVHVKPV
1051 FDRVFHSGHC TELKLESAVL LKEELAAKTV EYDKCIKSLY QPIRRNHQIA
1101 GEWSGMMLRL TGESHQPWSG LPMFAPSVVS SEGILGAIIN RLSNKGMKHH
1151 TVSLYLETNN QQPITEWVAT IDVDSNVERL AKVPLSQQIT KVQKLKVQYA
1201 NRAQPLYPEL EPLVRKVESL LEKFETLDET TVEKLMLVKI EGLYQGQPKS
1251 TLKIAMKKIY NLEKTEQQYA LAAMHQESHK GLELSTNVSY PKIGSPFRYD
1301 PTFYAEDERM NGTLIVKLQS PQEQVFHVKF QATKSEEQLK ETEYEWFEVR
1351 CLAEQKAGKI MTDACRKAVL KDNSLDQLKI AVTVPRNVHP KIQTLAYKTL
1401 DLMKYMWYPK MQTEVAGLKQ REVLQALQHT EREVRISVNA TRESLWHLLY
1451 DVRVEMPFEN VTFSKVNIPG VRPAHMQLTT KEQLEHVYYR GQKDNVCVLG
1501 DKSVRTYDNV TFGLDVKTGC EYVLTRDTSS GTPDFTVTFQ VVKPDTFAKK
1551 IRVQLENTLV ELEPFTTTDR YITVVVNGTQ YQITFEKPVV FEYAAGKRVF
1601 LNVVDTSNVH HAPVITLYTE PKEVRVFFDG HSAKVFVVNK YKGNTKGVCG
1651 NNDNEQAHEF IGPNGKEYQH ANEFIASYGI GQACKVPAEN TREKLMETLK
1701 KEVEQIRRQE LIKKEKLRKE MQELERARNP QWMEQQEEQF WGEPLSTVSN
1751 EEWTTDSVEE QQLQRQVLKT AMSIENGHIC FSARPIATCK QGYKNHGVLR
1801 TERVESICLE KNEEAAIQAV QEIRAGQVVN IKSLEPYQKG RLFTMHKVPR
1851 CERHD_PE_N Q_LIQ
Contig12013 refers to a contiguous sequence assembled from EST data produced by and held at Moredun Research Institute. It is not publically available:
BLAST search of translated contigs 12013:

**Sequences producing significant alignments:**

| **Accession** | **Description** | **Max score** | **Total score** | **Query coverage** | **E value** | **Links** |
|---|---|---|---|---|---|---|
| XP_002415224.1 | vitellogenin, putative [Ixodes scapularis] >gb\|EEC18889.1\| vitellogenin, putative [Ixodes scapularis] | 931 | 931 | 91% | 0.0 | 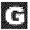 |
| AAW78557.2 | vitellogenin [Dermacentor variabilis] | 799 | 799 | 89% | 0.0 | |
| AAA92143.1 | GP80 precursor [Rhipicephalus microplus] | 520 | 520 | 64% | 8e-145 | |

NB GP80 has been tested as a vaccine candidate against the tick *Rhipicephalus (Boophilus) microplus* (Tellam et al., Vet Parasitol. 2002 Jan 3;103(1-2):141-56)
>JH 2
Match to: **contig00171** Score: **101** Expect: **6.4e-06**
**length=2053 numreads=831**
Translated in frame 1
Nominal mass (Mᵣ) : **78467;** Calculated pI value: **8.82**
NCBI BLAST search of contig00171 against nr
Unformatted sequence string for pasting into other applications
Variable modifications: Carbamidomethyl (C),Oxidation (M),Propionamide (C) Cleavage by Trypsin: cuts C-term side of KR unless next residue is P Number of mass values searched: **87**
Number of mass values matched: **23**
Sequence Coverage: **38%**
Matched peptides shown in **Bold**

1 QLKQQRLTQE QLVKEQAYPE YLRMIAYEDE YEKVTGRKPA TEKIEQALQR
51 IVKTTEKHWS QIEPELREKL NVNNAQVRSI EYIITAIGQK KQPIAITGHA
101 ILASTPSKQA RLAELTVDVE KHPISFEMQA VSAFAKAPQP FKAEIREQDQ
151 RGILGFVAQL ETPKIGKKQY AGKIEMTKSE EQKQLMKRSI QQQPWYYRQC
201 EEDKKEYTSE MSSACTRTRY HKSALNRVYA EIELPEEIPQ PIYNISRIIR
251 DTLKVKLYGN LHATYNRKDM QQNKLQVELV YIDRFPAMHV ANLTIRTPVN
301 EELHFERIAL PKALRPNSMW TLKEQIKAFL KNNRPEPVCV YNGKVIRTFD
351 NVTVSLDTVK VGQKYLVARD NDEQSKFSIV ATKVQAAEES QTVLEVLLRD
401 ATLIKLVPSV QKGVYKVHVN QTVTEVTPHK VQVYQYGPWA KHMLTLHVEE
451 HKEGQDTLVV KVRDMHVHIV YDGKNFLIEI AGPQIKGQLT GLCGDLNHQH
501 IDELTGPRGC HYEREEDFVR AFSFVPETNV AIEGEWVCPE GVHPRAASQM
551 QISKKMEQQQ AAQQKKQLNI QQRQIVREEY NKMTPETKMI HQDGKVCYSV
601 DPVQACKPGL RAVETEMHTV AFVCLPIQHP MTAMIQREIQ VHGVIRNIPS
651 VMPGALEHIL YHQIKTPISC AN_TANMGQI KLFS

**Sequences producing significant alignments:**

| **Accession** | **Description** | **Max score** | **Total score** | **Query coverage** | **E value** | **Links** |
|---|---|---|---|---|---|---|
| BAH02666.2 | vitellogenin [Ornithodoros moubata] | 310 | 310 | 91% | 6e-82 | |
| XP_002403966.1 | vitellogenin, putative [Ixodes scapularis] >gb\|EEC14774.1\| vitellogenin, putative [Ixodes scapularis] | 243 | 243 | 95% | 7e-62 | 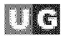 |
| ABW82681.2 | vitellogenin-2 precursor [Dermacentor variabilis] | 226 | 226 | 87% | 7e-57 | |
| BAE94323.1 | vitellogenin fused with superoxide dismutase [Daphnia magna] | 82.8 | 82.8 | 65% | 2e-13 | |
| BAD05137.1 | vitellogenin fused with superoxide dismutase [Daphnia magna] | 82.4 | 82.4 | 65% | 2e-13 | |
| ABS88989.1 | vitellogenin [Rhipicephalus microplus] | 79.0 | 79.0 | 48% | 2e-12 | |
| ADD73552.1 | vitellogenin 2 [Paracyclopina nana] | 79.0 | 79.0 | 61% | 3e-12 | |
| BAE94322.1 | vitellogenin fused with superoxide dismutase [Daphnia magna] | 79.0 | 79.0 | 65% | 3e-12 | |
| BAE94324.1 | vitellogenin fused with superoxide dismutase [Daphnia magna] | 79.0 | 79.0 | 65% | 3e-12 | |
| AAA92143.1 | GP80 precursor [Rhipicephalus microplus] | | | | | |

NB GP80 has been tested as a vaccine candidate against the tick *Rhipicephalus (Boophilus) microplus* (Tellam et al., Vet Parasitol. 2002 Jan 3;103(1-2):141-56)
>JH 3
Match to: **contig11897** Score: **112** Expect: **5.1e-07**
**length=3575 numreads=721**
Translated in frame 6
Nominal mass (Mᵣ): **134318;** Calculated pI value: **7.89**
NCBI BLAST search of contig11897 against nr
Unformatted sequence string for pasting into other applications
Variable modifications: Carbamidomethyl (C),Oxidation (M),Propionamide (C)
Cleavage by Trypsin: cuts C-term side of KR unless next residue is P
Number of mass values searched: **92**
Number of mass values matched: **30**
Sequence Coverage: **26%**
Matched peptides shown in **Bold**

1 SLVFLGAHPS NTASISTMRV LLTFVALAVA VSAAAYHGQK QVVIPDLFEG
51 QREYVVNYRS LVATGLPLQS QRFAGLEKYG ILTVQVHEQH GTTKVLGLRL
101 GHVTSGSFDK EVEDVENRGI EGISHSIKEI VEKYGAVFVT IQDNEVQKIK
151 VPQGMPEEIV NIYRGIAALF TVGKPDTHVH GTESHPFTYQ GEHGEQLPVV
201 YRRQEKGIAG NFETTYEILS NPEHEYGYLN VTKTRNYLKK VGPDGRFFQN
251 GHDAHGCGPV CLTHKPEQID QNMQPDTTAW ETPVTEGCPV KFHPKKDLVE
301 AFTTYNYNMT VEQGGKIAVI HEAKGLDKKV LPLRKQQILT VSLLKVTLVE
351 VRPISQIWEE KAATKQYSDI TYRFPEGHKH DLAYLSLYSK GSKGEIVTEQ
401 ILPMLKALSK IIVSDNIELK SQTGDKVVQL TQALGVLTKT ELQSIWTVIG
451 EPVSEKTATE FEKVQRKVLV DIIALSGSND AAEFLVELIQ QERLTVLETV
501 HTLETLQKSI VKPTLTIIKQ LLNVCTETKF QKTRVVFSTA CIAFSEIVRH
551 NCEDYVVSQM TEAPKMQGEE KKHTVHSCGP HEYKAFIETI REKLYAAQEY
601 PQQVVYIQVL GRLAHPEALK ALVPYIYGEH EVIAQVEKMT RDNEHEDNSE
651 YVQFLRQVAI FALHHSVRKH GASVQPIVQG VYFNKKEDYE LRIAALSVLL
701 ATQPTEATFG RIVTELYKED NLEIASYTYS ALHALANSTL PCMKQSARRV
751 QNVLGAFPKK SYGIDYSKWG THTKYSPLMN FGYKGHWEIT QSNVSAIPRA
801 IYVGATANKG PFISTLGEFG MISKGLENLD RFVGQKGGVQ KIMENVMQRI
851 RRDARTFVGD ASVGRMLEEI ENAFDFNTEE NNEQLRAVVF GNVLGNEFYL
901 PVDKQFVTKV AEKVGEEMIK ILREEGSEKT LRYVRVLLPR TYVQVAPAVN
951 GLPVLMINRH PIVVSLALKD LKIRLGAEKE ELTLNPLTFA ASGLIQPTVY
1001 FTAFHTAMTI NPLETSHVGY GLRTIEQTYM SLPIDASIQY THQTKTLAFT
1051 FRPRFEKIFF HKTRAMTFKT EVLLISDVER PILDQYTVIK TQRKPYAIDR
1101 VFGEKLGMAL RVQGLTVNEN YADNLIYKIF TGQHTAVTSI LKGIANEWVL
1151 PRAWTVRIEQ NKQTPIDKVK VVLRLGDYLK DRMLTEQTEQ K

**Sequences producing significant alignments:**

| **Accession** | **Description** | **Max score** | **Total score** | **Query coverage** | **E value** | **Links** |
|---|---|---|---|---|---|---|
| BAH02666.2 | vitellogenin [Ornithodoros moubata] | 686 | 686 | 97% | 0.0 | |
| ABW82681.2 | vitellogenin-2 precursor [Dermacentor variabilis] | 657 | 657 | 95% | 0.0 | |
| XP_002403966.1 | vitellogenin, putative [Ixodes scapularis] >gb\|EEC14774.1\| vitellogenin, putative [Ixodes scapularis] | 418 | 521 | 73% | 3e-114 | 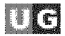 |
| XP_002415224.1 | vitellogenin, putative [Ixodes scapularis] >gb\|EEC18889.1\| vitellogenin, putative [Ixodes scapularis] | 246 | 246 | 87% | 1e-62 | 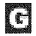 |
| AAW78557.2 | vitellogenin [Dermacentor variabilis] | 208 | 208 | 77% | 5e-51 | |
| XP_002741413.1 | PREDICTED: VITellogenin structural genes (yolk protein genes) family member (vit-1)-like [Saccoglossus kowalevskii] | 128 | 128 | 83% | 5e-27 | 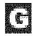 |
| AAA92143.1 | GP80 precursor [Rhipicephalus microplus] | | | | | |

NB GP80 has been tested as a vaccine candidate against the tick *Rhipicephalus (Boophilus) microplus* (Tellam et al., Vet Parasitol. 2002 Jan 3;103(1-2):141-56).

### Appendices:

Appendix 1: Mite counts of vaccinated hens (Group 2) versus control hens (Group 1) for each individual cage.

| **25/11/2010** | | | | **10/12/2010** | | | | **11/01/2011** | | | | **28/01/2011** | | | |
|---|---|---|---|---|---|---|---|---|---|---|---|---|---|---|---|
| **Group 1** | | **Group 2** | | **Group 1** | | **Group 2** | | **Group 1** | | **Group 2** | | **Group 1** | | **Group 2** | |
| **Cage No** | **Mite No** | **Cage No** | **Mite No** | **Cage No** | **Mite No** | **Cage No** | **Mite No** | **Cage No** | **Mite No** | **Cage No** | **Mite No** | **Cage No** | **Mite No** | **Cage No** | **Mite No** |
| **3** | 1 | **1** | 0 | **3** | 0 | **1** | 0 | **3** | 2 | **1** | 1 | **3** | 19 | **1** | 143 |
| **4** | 1 | **2** | 2 | **4** | 0 | **2** | 0 | **4** | 6 | **2** | 0 | **4** | 144 | **2** | 7 |
| **7** | 0 | **5** | 2 | **7** | 0 | **5** | 0 | **7** | 4 | **5** | 2 | **7** | 90 | **5** | 183 |
| **8** | 3 | **6** | 4 | **8** | 0 | **6** | 0 | **8** | 1 | **6** | 70 | **8** | 5 | **6** | 134 |
| **11** | 0 | **9** | 1 | **11** | 0 | **9** | 0 | **11** | 0 | **9** | 0 | **11** | 0 | **9** | 1 |
| **12** | 1 | **10** | 1 | **12** | 0 | **10** | 0 | **12** | 0 | **10** | 4 | **12** | 0 | **10** | 1 |
| **15** | 3 | **13** | 2 | **15** | 0 | **13** | 1 | **15** | 0 | **13** | 218 | **15** | 3 | **13** | 130 |
| **16** | 0 | **14** | 0 | **16** | 0 | **14** | 0 | **16** | 3 | **14** | 1 | **16** | 1 | **14** | **6** |
| **17** | 1 | **19** | 0 | **17** | 0 | **19** | 0 | **17** | 1 | **19** | 0 | **17** | 11 | **19** | 0 |
| **18** | 0 | **20** | 4 | **18** | 0 | **20** | 0 | **18** | 1 | **20** | 0 | **18** | 3 | **20** | 121 |
| **21** | 2 | **23** | 3 | **21** | 0 | **23** | 0 | **21** | 0 | **23** | 0 | **21** | 4 | **23** | 110 |
| **22** | 1 | **24** | 0 | **22** | 0 | **24** | 0 | **22** | 9 | **24** | 0 | **22** | 1 | **24** | 3 |
| **25** | 1 | **27** | 1 | **25** | 0 | **27** | 0 | **25** | 1 | **27** | 0 | **25** | 1 | **27** | 1 |
| **26** | 0 | **28** | 5 | **26** | 0 | **28** | 0 | **26** | 0 | **28** | 23 | **26** | 0 | **28** | 411 |
| **29** | 0 | **31** | 2 | **29** | 0 | **31** | 0 | **29** | 0 | **31** | 0 | **29** | 198 | **31** | 244 |
| **30** | 0 | **32** | 0 | **30** | 0 | **32** | 0 | **30** | 1 | **32** | 1 | **30** | 2 | **32** | 26 |
| **35** | 0 | **33** | 0 | **35** | 0 | **33** | 0 | **35** | 0 | **33** | 1 | **35** | 1 | **33** | 2 |
| **36** | 0 | **34** | 1 | **36** | 0 | **34** | 0 | **36** | 0 | **34** | 0 | **36** | 1 | **34** | 1 |
| **39** | 0 | **37** | 5 | **39** | 0 | **37** | 0 | **39** | 0 | **37** | 1 | **39** | 0 | **37** | 7 |
| **40** | 1 | **38** | 0 | **40** | 0 | **38** | 0 | **40** | 0 | **38** | 0 | **40** | 0 | **38** | 0 |
| **43** | 0 | **41** | 0 | **43** | 0 | **41** | 0 | **43** | 0 | **41** | 0 | **43** | 0 | **41** | 3 |
| **44** | 1 | **42** | 1 | **44** | 0 | **42** | 0 | **44** | 0 | **42** | 0 | **44** | 0 | **42** | 11 |
| **47** | 0 | **45** | 1 | **47** | 0 | **45** | 0 | **47** | 0 | **45** | 25 | **47** | 4 | **45** | 35 |
| **48** | 1 | **46** | 1 | **48** | 0 | **46** | 0 | **48** | 0 | **46** | 1 | **48** | 1 | **46** | 2 |
| **49** | 0 | **51** | 0 | **49** | 0 | **51** | 0 | **49** | 6 | **51** | 16 | **49** | 12 | **51** | 5 |
| **50** | 0 | **52** | 1 | **50** | 0 | **52** | 0 | **50** | 6 | **52** | 38 | **50** | 6 | **52** | 46 |
| **53** | 0 | **55** | 0 | **53** | 0 | **55** | 0 | **53** | 0 | **55** | 11 | **53** | 12 | **55** | 18 |
| **54** | 0 | **56** | 2 | **54** | 0 | **56** | 0 | **54** | 0 | **56** | 3 | **54** | 56 | **56** | 2 |
| **57** | 2 | **59** | 1 | **57** | 0 | **59** | 0 | **57** | 10 | **59** | 32 | **57** | 27 | **59** | 148 |
| **58** | 1 | **60** | 0 | **58** | 0 | **60** | 0 | **58** | 5 | **60** | 68 | **58** | 8 | **60** | 291 |
| **61** | 0 | **63** | 0 | **61** | 0 | **63** | 0 | **61** | 25 | **63** | 19 | **61** | 401 | **63** | 7 |
| **62** | 3 | **64** | 0 | **62** | 0 | **64** | 1 | **62** | 10 | **64** | 5 | **62** | 37 | **64** | 12 |
| **67** | 3 | **65** | 1 | **67** | 0 | **65** | 0 | **67** | 4 | **65** | 0 | **67** | 44 | **65** | 2 |
| **68** | 5 | **66** | 0 | **68** | 0 | **66** | 0 | **68** | 2 | **66** | 2 | **68** | 4 | **66** | 27 |
| **71** | 0 | **69** | 4 | **71** | 0 | **69** | 0 | **71** | 0 | **69** | 156 | **71** | 34 | **69** | 318 |
| **72** | 1 | **70** | 3 | **72** | 0 | **70** | 0 | **72** | 0 | **70** | 25 | **72** | 2 | **70** | 82 |
| **75** | 5 | **73** | 3 | **75** | 0 | **73** | 1 | **75** | 3 | **73** | 2 | **75** | 25 | **73** | 30 |
| **76** | 2 | **74** | 0 | **76** | 0 | **74** | 0 | **76** | 0 | **74** | 1 | **76** | 2 | **74** | 1 |
| **79** | 0 | **77** | 1 | **79** | 0 | **77** | 0 | **79** | 1 | **77** | 20 | **79** | 11 | **77** | 91 |
| **80** | | **78** | 0 | **80** | 2 | **78** | 0 | **80** | | **78** | 16 | **80** | 31 | **78** | 8 |
| **81** | | **83** | 2 | **81** | 0 | **83** | 0 | **81** | 0 | **83** | 0 | **81** | 1 | **83** | 7 |
| **82** | 0 | **84** | 2 | **82** | 2 | **84** | 0 | **82** | 0 | **84** | 4 | **82** | 2 | **84** | 96 |
| **85** | 0 | **87** | 0 | **85** | 0 | **87** | 0 | **85** | 0 | **87** | 0 | **85** | 0 | **87** | 1 |
| **86** | 0 | **88** | 3 | **86** | 0 | **88** | 0 | **86** | 2 | **88** | 24 | **86** | 3 | **88** | 54 |
| **89** | **2** | **91** | 1 | **89** | 0 | **91** | 0 | **89** | 0 | **91** | 2 | **89** | 0 | **91** | 2 |
| **90** | 0 | **92** | 0 | **90** | 0 | **92** | 0 | **90** | 4 | **92** | 7 | **90** | 1 | **92** | 197 |
| **93** | 1 | **95** | 0 | **93** | 0 | **95** | 0 | **93** | 0 | **95** | 0 | **93** | 1 | **95** | 1 |
| **94** | 1 | **96** | 0 | **94** | 0 | **96** | 0 | **94** | 0 | **96** | 0 | **94** | 1 | **96** | 8 |
| **99** | 3 | **97** | 0 | **99** | 0 | **97** | 0 | **99** | 160 | **97** | 5 | **99** | 181 | **97** | 10 |
| **100** | 0 | **98** | 0 | **100** | 0 | **98** | 0 | **100** | 0 | **98** | 6 | **100** | 72 | **98** | 1 |
| **103** | 0 | **101** | 3 | **103** | 0 | **101** | 0 | **103** | 0 | **101** | 11 | **103** | 10 | **101** | 81 |
| **104** | 0 | **102** | 1 | **104** | 0 | **102** | 0 | **104** | 1 | **102** | 25 | **104** | 59 | **102** | 75 |
| **107** | 0 | **105** | 0 | **107** | 0 | **105** | 0 | **107** | 7 | **105** | 0 | **107** | 19 | **105** | 16 |
| **108** | 0 | **106** | 0 | **108** | 0 | **106** | 0 | **108** | 6 | **106** | 0 | **108** | 55 | **106** | 10 |
| **111** | 2 | **109** | 0 | **111** | 0 | **109** | 0 | **111** | 82 | **109** | 184 | **111** | 43 | **109** | 121 |
| **112** | 2 | **110** | 1 | **112** | 0 | **110** | 0 | **112** | 224 | **110** | 58 | **112** | 141 | **110** | 55 |
| **114** | 1 | **115** | 0 | **114** | 0 | **115** | 0 | **114** | 2 | **115** | 0 | **114** | 10 | **115** | 87 |
| **117** | 2 | **116** | 0 | **117** | 0 | **116** | 0 | **117** | 0 | **116** | 0 | **117** | 5 | **116** | 23 |
| **118** | 0 | **119** | 1 | **118** | 0 | **119** | 0 | **118** | 3 | **119** | 1 | **118** | 31 | **119** | 0 |
| **118** | 1 | **120** | 1 | **118** | 0 | **120** | 0 | **118** | 0 | **120** | 1 | **118** | 53 | **120** | 80 |
| **121** | 0 | **123** | 1 | **121** | 0 | **123** | 0 | **121** | 3 | **123** | 0 | **121** | 18 | **123** | 23 |
| **122** | 0 | **124** | 1 | **122** | 0 | **124** | 0 | **122** | 0 | **124** | 137 | **122** | 83 | **124** | 260 |
| **125** | 0 | **127** | 0 | **125** | 0 | **127** | 0 | **125** | 1 | **127** | 0 | **125** | 7 | **127** | 3 |
| **126** | 0 | **128** | 0 | **126** | 0 | **128** | 0 | **126** | 0 | **128** | 0 | **126** | 47 | **128** | 1 |
| **131** | 0 | **129** | 0 | **131** | 0 | **129** | 0 | **131** | 0 | **129** | 0 | **131** | 1 | **129** | 0 |
| **132** | 1 | **130** | 0 | **132** | 0 | **130** | 0 | **132** | 1 | **130** | 1 | **132** | 0 | **130** | 1 |
| **135** | 0 | **133** | 0 | **135** | 0 | **133** | 0 | **135** | 0 | **133** | 0 | **135** | 1 | **133** | 0 |
| **136** | 1 | **134** | 4 | **136** | 0 | **134** | 0 | **136** | 0 | **134** | 0 | **136** | 20 | **134** | 0 |
| **139** | 0 | **137** | 0 | **139** | 0 | **137** | 0 | **139** | 0 | **137** | 0 | **139** | 20 | **137** | 1 |
| **140** | 0 | **138** | 1 | **140** | 0 | **138** | 0 | **140** | 0 | **138** | 0 | **140** | 1 | **138** | 0 |
| **143** | 0 | **141** | 1 | **143** | 0 | **141** | 0 | **143** | 0 | **141** | 6 | **143** | 0 | **141** | 135 |
| **144** | 0 | **142** | 1 | **144** | 0 | **142** | 0 | **144** | 0 | **142** | 0 | **144** | 0 | **142** | 59 |
| **145** | 0 | **147** | 1 | **145** | 0 | **147** | 0 | **145** | 0 | **147** | 6 | **145** | 10 | **147** | 68 |
| **146** | 0 | **148** | 1 | **146** | 0 | **148** | 1 | **146** | 0 | **148** | 127 | **146** | 3 | **148** | 162 |
| **149** | 1 | **151** | 1 | **149** | 0 | **151** | 0 | **149** | 1 | **151** | 0 | **149** | 310 | **151** | 19 |
| **150** | 0 | **152** | 0 | **150** | 0 | **152** | 0 | **150** | 1 | **152** | 0 | **150** | 124 | **152** | 1 |
| **153** | 0 | **155** | 3 | **153** | 0 | **155** | 0 | **153** | 0 | **155** | 51 | **153** | 51 | **155** | 14 |
| **154** | 0 | **156** | 1 | **154** | 0 | **156** | 0 | **154** | 0 | **156** | 7 | **154** | 35 | **156** | 221 |
| **157** | 1 | **159** | 0 | **157** | 0 | **159** | 0 | **157** | 0 | **159** | 0 | **157** | 43 | **159** | 11 |
| **158** | 1 | **160** | 0 | **158** | 0 | **160** | 0 | **158** | 4 | **160** | 7 | **158** | 141 | **160** | 17 |
| **163** | 0 | **161** | 2 | **163** | 0 | **161** | 0 | **163** | 0 | **161** | 0 | **163** | 3 | **161** | 9 |
| **164** | 0 | **162** | 0 | **164** | 0 | **162** | 0 | **164** | 0 | **162** | 0 | **164** | 0 | **162** | 0 |
| **167** | 1 | **165** | 2 | **167** | 2 | **165** | 0 | **167** | 1 | **165** | 6 | **167** | 2 | **165** | 238 |
| **168** | 0 | **166** | 2 | **168** | 0 | **166** | 0 | **168** | 0 | **166** | 0 | **168** | 0 | **166** | 0 |
| **171** | 0 | **169** | 0 | **171** | 0 | **169** | 0 | **171** | 1 | **169** | 0 | **171** | 45 | **169** | 8 |
| **172** | 3 | **170** | 0 | **172** | 0 | **170** | 0 | **172** | 2 | **170** | 0 | **172** | 5 | **170** | 5 |
| **175** | 5 | **173** | 1 | **175** | 1 | **173** | 0 | **175** | 1 | **173** | 191 | **175** | 16 | **173** | 225 |
| **176** | 1 | **174** | 2 | **176** | 0 | **174** | 0 | **176** | 0 | **174** | 1 | **176** | 1 | **174** | 184 |
| **177** | 1 | **179** | 6 | **177** | 0 | **179** | 0 | **177** | 0 | **179** | 0 | **177** | 30 | **179** | 32 |
| **178** | 0 | **180** | 0 | **178** | 0 | **180** | 0 | **178** | 0 | **180** | 0 | **178** | 10 | **180** | 60 |
| **181** | 0 | **183** | 0 | **181** | 0 | **183** | 0 | **181** | 0 | **183** | 0 | **181** | 91 | **183** | 1 |
| **182** | 1 | **184** | 0 | **182** | 0 | **184** | 0 | **182** | 7 | **184** | 0 | **182** | 0 | **184** | 0 |
| **185** | 0 | **187** | 0 | **185** | 0 | **187** | 0 | **185** | 0 | **187** | 0 | **185** | 0 | **187** | 39 |
| **186** | 0 | **188** | 0 | **186** | 0 | **188** | 0 | **186** | 0 | **188** | 0 | **186** | 1 | **188** | 10 |
| **189** | 0 | **191** | 0 | **189** | 0 | **191** | 0 | **189** | 0 | **191** | 0 | **189** | 0 | **191** | 0 |
| **190** | 0 | **192** | 0 | **190** | 0 | **192** | 0 | **190** | 4 | **192** | 0 | **190** | 0 | **192** | 0 |
| | 0.76 | | 1.02 | | 0.073 | | 0.04 | | 6.516 | | 17 | | 31.3 | | 56.27 |
| | 71.8 | | 99 | | 7.073 | | 4.04 | | 625.5 | | 1647 | | 3039 | | 5458 |

| **15/02/2011** | | | | **01/03/2011** | | | | **15/03/2011** | | | |
|---|---|---|---|---|---|---|---|---|---|---|---|
| **Group 1** | | **Group 2** | | **Group 1** | | **Group 2** | | **Group 1** | | **Group 2** | |
| **Cage No** | **Mite No** | **Cage No** | **Mite No** | **Cage No** | **Mite No** | **Cage No** | **Mite No** | **Cage No** | **Mite No** | **Cage No** | **Mite No** |
| **3** | 1210 | **1** | 217 | **3** | 2541 | **1** | 388 | **3** | 1063 | **1** | 101 |
| **4** | 607 | **2** | 180 | **4** | 2854 | **2** | 398 | 4 | 992 | **2** | 81 |
| **7** | 26 | **5** | 964 | **7** | 156 | **5** | 1100 | 7 | 40 | **5** | 560 |
| **8** | 182 | **6** | 575 | **8** | 920 | **6** | 572 | **8** | 1005 | **6** | 204 |
| **11** | 839 | **9** | 138 | **11** | 1160 | **9** | 223 | **11** | 1033 | **9** | 63 |
| **12** | 331 | **10** | 830 | **12** | 1031 | **10** | 613 | **12** | 1060 | **10** | 240 |
| **15** | 88 | **13** | 1362 | **15** | 173 | **13** | 1060 | **15** | 130 | **13** | 320 |
| **16** | 500 | **14** | 174 | **16** | 768 | **14** | 42 | **16** | 682 | **14** | 75 |
| **17** | 75 | **19** | 148 | **17** | 444 | **19** | 522 | **17** | 235 | **19** | 404 |
| **18** | 76 | **20** | 586 | **18** | 580 | **20** | 1923 | **18** | 140 | **20** | 520 |
| **21** | 212 | **23** | 361 | **21** | 2235 | **23** | 472 | **21** | 1042 | **23** | 440 |
| **22** | 350 | **24** | 62 | **22** | 1383 | **24** | 782 | **22** | 960 | **24** | 208 |
| **25** | 11 | **27** | 440 | **25** | 540 | **27** | 362 | **25** | 350 | **27** | 240 |
| **26** | 91 | **28** | 871 | **26** | 1528 | **28** | 1020 | **26** | 600 | **28** | 1060 |
| **29** | 513 | **31** | 526 | **29** | 2842 | **31** | 748 | **29** | 5362 | **31** | 224 |
| **30** | 172 | **32** | 121 | **30** | 872 | **32** | 1050 | **30** | 920 | **32** | 280 |
| **35** | 1 | **33** | 18 | **35** | 92 | **33** | 464 | **35** | 520 | **33** | 322 |
| **36** | 1 | **34** | 48 | **36** | 1184 | **34** | 1149 | **36** | 780 | **34** | 336 |
| **39** | 1 | **37** | 224 | **39** | 6 | **37** | 652 | **39** | 280 | **37** | 1032 |
| **40** | 0 | **38** | 4 | **40** | 276 | **38** | 10 | **40** | 2804 | **38** | 44 |
| **43** | 300 | **41** | 75 | **43** | 691 | **41** | 73 | **43** | 880 | **41** | 284 |
| **44** | 34 | **42** | 381 | **44** | 530 | **42** | 112 | **44** | 2086 | **42** | 272 |
| **47** | 112 | **45** | 803 | **47** | 206 | **45** | 1200 | **47** | 63 | **45** | 408 |
| **48** | 2 | **46** | 5 | **48** | 708 | **46** | 88 | **48** | 995 | **46** | 140 |
| **49** | 863 | **51** | 21 | **49** | 361 | **51** | 196 | **49** | 776 | **51** | 40 |
| **50** | 215 | **52** | 40 | **50** | 628 | **52** | 784 | **50** | 744 | **52** | 130 |
| **53** | 41 | **55** | 60 | **53** | 2190 | **55** | 264 | **53** | 2628 | **55** | 93 |
| **54** | 490 | **56** | 38 | **54** | 2448 | **56** | 416 | **54** | 2024 | **56** | 276 |
| **57** | 431 | **59** | 119 | **57** | 2568 | **59** | 740 | **57** | 2040 | **59** | 144 |
| **58** | 211 | **60** | 78 | **58** | 226 | **60** | 1592 | **58** | 952 | **60** | 664 |
| **61** | 665 | **63** | 63 | **61** | 2000 | **63** | 146 | **61** | 2890 | **63** | 131 |
| **62** | 484 | **64** | 150 | **62** | 1184 | **64** | 123 | **62** | 1030 | **64** | 172 |
| **67** | 579 | **65** | 3 | **67** | 1728 | **65** | 336 | **67** | 940 | **65** | 296 |
| **68** | 95 | **66** | 61 | **68** | 2834 | **66** | 1301 | **68** | 1432 | **66** | 224 |
| **71** | 340 | **69** | 27 | **71** | 1368 | **69** | 1776 | **71** | 1820 | **69** | 895 |
| **72** | 16 | **70** | 29 | **72** | 296 | **70** | 676 | **72** | 800 | **70** | 406 |
| **75** | 102 | **73** | 23 | **75** | 960 | **73** | 984 | **75** | 1808 | **73** | 452 |
| **76** | 196 | **74** | 32 | **76** | 2652 | **74** | 796 | **76** | 3192 | **74** | 310 |
| **79** | 146 | **77** | 51 | **79** | 1808 | **77** | 853 | **79** | 1596 | **77** | 286 |
| **80** | 591 | **78** | 26 | **80** | 944 | **78** | 1116 | **80** | 3024 | **78** | 151 |
| **81** | 40 | **83** | 23 | **81** | 1576 | **83** | 610 | **81** | 982 | **83** | 110 |
| **82** | 14 | **84** | 53 | **82** | 684 | **84** | 1842 | **82** | 1400 | **84** | 468 |
| **85** | 8 | **87** | 8 | **85** | 2992 | **87** | 596 | **85** | 3064 | **87** | 896 |
| **86** | 71 | **88** | 32 | **86** | 1360 | **88** | 368 | **86** | 1640 | **88** | 200 |
| **89** | 17 | **91** | 5 | **89** | 1416 | **91** | 130 | **89** | 1600 | **91** | 96 |
| **90** | 211 | **92** | 89 | **90** | 1280 | **92** | 450 | **90** | 2040 | **92** | 788 |
| **93** | 407 | **95** | 1 | **93** | 700 | **95** | 568 | **93** | 1010 | **95** | 212 |
| **94** | 4 | **96** | 3 | **94** | 121 | **96** | 576 | **94** | 688 | **96** | 69 |
| **99** | 432 | **97** | 95 | **99** | 720 | **97** | 1178 | **99** | 1862 | **97** | 431 |
| **100** | 870 | **98** | 314 | **100** | 880 | **98** | 473 | **100** | 3400 | **98** | 300 |
| **103** | 252 | **101** | 113 | **103** | 264 | **101** | 400 | **103** | 1020 | **101** | 903 |
| **104** | 275 | **102** | 124 | **104** | 1338 | **102** | 183 | **104** | 960 | **102** | 660 |
| **107** | 583 | **105** | 44 | **107** | 2112 | **105** | 900 | **107** | 3065 | **105** | 220 |
| **111** | 882 | **106** | 12 | **111** | 948 | **106** | 621 | **111** | 1744 | **106** | 180 |
| **112** | 653 | **109** | 143 | **112** | 936 | **109** | 1036 | **112** | 784 | **109** | 310 |
| **113** | 330 | 110 | 93 | **113** | 1084 | 110 | 928 | **113** | 2456 | **110** | 276 |
| **114** | 468 | **115** | 107 | **114** | 2024 | **115** | 550 | **114** | 1050 | **115** | 584 |
| **117** | 225 | **116** | 196 | **117** | 3612 | **116** | 2432 | **117** | 4620 | **116** | 798 |
| **118** | 598 | **119** | 44 | **118** | 2576 | **119** | 868 | **118** | 1652 | **119** | 240 |
| **118** | 425 | **120** | 31 | **118** | 2075 | **120** | 1880 | **108** | 852 | **120** | 364 |
| **121** | 198 | **123** | 326 | **121** | 944 | **123** | 525 | **121** | 1160 | **123** | 224 |
| **122** | 193 | **124** | 77 | **122** | 1795 | **124** | 1680 | **122** | 2000 | **124** | 968 |
| **125** | 300 | **127** | 393 | **125** | 1340 | **127** | 328 | **125** | 320 | **127** | 194 |
| **126** | 388 | **128** | 16 | **126** | 2640 | **128** | 648 | **126** | 1022 | **128** | 336 |
| **131** | 1029 | **129** | 27 | **131** | 1324 | **129** | 608 | **131** | 2354 | **129** | 140 |
| **132** | 53 | **130** | 199 | **132** | 218 | **130** | 820 | **132** | 3025 | **130** | 320 |
| **135** | 5 | **133** | 10 | **135** | 1056 | **133** | 1127 | **135** | 712 | **133** | 736 |
| **136** | 110 | **134** | 46 | **136** | 880 | **134** | 113 | **136** | 844 | **134** | 184 |
| **139** | 713 | **137** | 15 | **139** | 1568 | **137** | 809 | **139** | 2513 | **137** | 630 |
| **140** | 8 | **138** | 57 | **140** | 3640 | **138** | 1048 | **140** | 2904 | **138** | 284 |
| **143** | 11 | **141** | 18 | **143** | 712 | **141** | 1868 | **143** | 1056 | **141** | 432 |
| **144** | 28 | **142** | 16 | **144** | 764 | **142** | | **144** | 452 | **142** | 228 |
| **145** | 586 | **147** | 769 | **145** | 2106 | **147** | 1929 | **145** | 1088 | **147** | 233 |
| **146** | 467 | **148** | 450 | **146** | 2084 | **148** | 1682 | **146** | 1236 | **148** | 410 |
| **149** | 1299 | **151** | 800 | **149** | 2792 | **151** | 1288 | **149** | 2981 | **151** | 312 |
| **150** | 528 | **152** | 150 | **150** | 1952 | **152** | 608 | **150** | 3120 | **152** | 97 |
| **153** | 362 | **155** | 230 | **153** | 1384 | **155** | 512 | **153** | 1250 | **155** | 130 |
| **154** | 641 | **156** | 1232 | **154** | 920 | **156** | 1724 | **154** | 880 | **156** | 400 |
| **157** | 2622 | **159** | 386 | **157** | 3056 | **159** | 752 | **157** | 3520 | **159** | 110 |
| **158** | 810 | **160** | 500 | **158** | 1250 | **160** | 1180 | **158** | 2368 | **160** | 604 |
| **163** | 442 | **161** | 291 | **163** | 1835 | **161** | 660 | **163** | 3358 | **161** | 648 |
| **164** | 257 | **162** | 454 | **164** | 2115 | **162** | 1357 | **164** | 3872 | **162** | 700 |
| **167** | 253 | **165** | 1164 | **167** | 1776 | **165** | 1513 | **167** | 1018 | **165** | 631 |
| **168** | 324 | **166** | 50 | **168** | 1584 | **166** | 520 | **168** | 1792 | **166** | 140 |
| **171** | **608** | **169** | 155 | **171** | 3104 | **169** | 1340 | **171** | 4362 | **169** | 300 |
| **172** | **662** | **170** | 903 | **172** | 106 | **170** | 1231 | **172** | 5104 | **170** | 410 |
| **175** | **402** | **173** | 870 | **175** | 1888 | **173** | 1155 | **175** | 1542 | **173** | 1520 |
| **176** | **484** | **174** | 216 | **176** | 1104 | **174** | 1968 | **176** | 963 | **174** | 608 |
| **177** | 15 | **179** | 278 | **177** | 644 | **179** | 740 | **177** | 1020 | **179** | 135 |
| **178** | 76 | **180** | 312 | **178** | 1056 | **180** | 793 | **178** | 1360 | **180** | 556 |
| **181** | 543 | **183** | 14 | **181** | 1156 | **183** | 1200 | **181** | 5266 | **183** | 504 |
| **182** | 23 | **184** | 43 | **182** | 243 | **184** | 186 | **182** | 3800 | **184** | 280 |
| **185** | 1 | **187** | 280 | **185** | 588 | **187** | 484 | **185** | 680 | **187** | 452 |
| **186** | 2 | **188** | 126 | **186** | 624 | **188** | 524 | **186** | 624 | **188** | 424 |
| **189** | 5 | **191** | 6 | **189** | 912 | **191** | 564 | **189** | 2086 | **191** | 86 |
| **190** | 5 | **192** | 4 | **190** | 844 | **192** | 328 | **190** | 1190 | **192** | 160 |
| | 337.7 | | 232.8 | | 1340 | | 816.03 | | 1671 | | 366.2 |
| | 32755 | | 22580 | | 1E+05 | | 78339 | | 2E+05 | | 35525 |

### References

1. Chauve, C., 1998. The poultry red mite Dermanyssus gallinae (De Geer, 1778): current situation and future prospects for control. Veterinary Parasitology, 79, 239-245.
2. Van Emous R., 2005. Wage war against the red mite! Poultry International 44: 26-33
3. Marangi M, Cafiero MA, Capelli G, Camarda A, Sparagano OA, Giangaspero A., 2009. Evaluation of the poultry red mite, Dermanyssus gallinae (Acari: Dermanyssidae) susceptibility to some acaricides in field populations from Italy. Exp Appl Acarol. 48:11-18.
4. Valiente Moro C, De Luna CJ, Tod A, Guy JH, Sparagano OA, Zenner L., 2009. The poultry red mite (Dermanyssus gallinae): a potential vector of pathogenic agents. Exp Appl Acarol. 93-104
5. Guy, J. H., Khajavi, M., Hlalel, M. M., and Sparagano, O., 2004, Red mite (Dermanyssus gallinae) prevalence in laying units in Northern England, Br.Poult.Sci., 45 Suppl 1, S15 - S16.
6. de la Fuente, J. & Kocan, K.M., 2003. Advances in the identification and characterisation of protective antigens for recombinant vaccines against tick infestations. Expert Rev Vaccines 2; 583-593.
7. Willadsen, P., 2004. Anti-tick vaccines. Parasitology 129 (Supplement); S367-S387.
8. Wright, H.W., Bartley, K., Nisbet, A.J., McDevitt, R., Sparks, N., Brocklehurst, S., Huntley, J.F., 2009. The testing of antibodies raised against poultry red mite antigens in an in vitro feeding assay; preliminary screen for vaccine candidates. Experimental and Applied Acarology 48, 81-91.
9. Bartley, K., Nisbet, A.J., Offer, J., Sparks, N., Wright, H, Huntley, J.F., 2009. Histamine Release Factor from Dermanyssus gallinae (De Geer): Characterisation and in vitro assessment as a protective antigen. Int. J. Parasitol. 39: 447-456 doi:10.1016/j.ijpara.2008.09.006
10. Harrington D, Canales M, de la Fuente J, de Luna C, Robinson K, Guy J, Sparagano O., 2009. Immunisation with recombinant proteins subolesin and Bm86 for the control of Dermanyssus gallinae in poultry. Vaccine. 27; 4056-4063.
11. Harrington D, Din HM, Guy J, Robinson K, Sparagano O., 2009. Characterization of the immune response of domestic fowl following immunization with proteins extracted from Dermanyssus gallinae. Vet Parasitol 160:285-294.
12. Cafiero MA, Galante D, Camarda A, Giangaspero A, Sparagano O., 2011. Why dermanyssosis should be listed as an occupational hazard. Occup Environ Med. 68:628.

## Claims

1. One or more antigens for use in a method of raising an immune response in an avian species wherein said one or more antigens comprises SEQ ID NO:1, or is at least 99% identical to SEQ ID NO: 1.

2. A purified or recombinant antigen or composition for use in a method of raising an immune response in an avian species, the antigen or composition comprising one or more *D. gallinae* antigens comprising the sequence:
(a) SEQ ID NO: 1;
or
(b) a sequence at least 99% identical to (a).

3. An immunogenic composition comprising a *D. gallinae* purified or recombinant antigen comprising the sequence:
(a) SEQ ID NO: 1;
or
(b) a sequence at least 99% identical to (a).

4. An isolated nucleic acid encoding the sequence:
(a) SEQ ID NO: 1;
or
(b) a sequence at least 99% identical to (a).
for use in a method of raising an immune response in an avian species.

5. A vector comprising the isolated nucleic acid according to claim 4.

## Patentansprüche

1. Ein oder mehrere Antigene zur Verwendung in einem Verfahren zum Erzeugen einer Immunantwort in einer Vogelart, wobei das eine oder die mehreren Antigene SEQ ID No: 1 umfassen oder mindestens zu 99% identisch mit SEQ ID No: 1 sind.

2. Gereinigtes oder rekombinantes Antigen oder Zusammensetzung zur Verwendung in einem Verfahren zum Erzeugen einer Immunantwort in einer Vogelart, wobei das Antigen oder die Zusammensetzung ein oder mehrere *D. Gallinae*-Antigene umfasst, umfassend die Sequenz:
(a) SEQ ID No: 1
oder
(b) eine mindestens zu 99% identische Sequenz zu (a).

3. Immunogene Zusammensetzung, umfassend ein gereinigtes oder rekombinantes *D. Gallinae*-Antigen, umfassend die Sequenz:
(a) SEQ ID No: 1
oder
(b) eine mindestens zu 99% identische Sequenz zu (a).

4. Isolierte Nucleinsäure, codierend die Sequenz:
(a) die SEQ ID No: 1;
oder
(b) eine mindestens zu 99% identische Sequenz zu (a),
zur Verwendung in einem Verfahren zum Erzeugen einer Immunantwort in einer Vogelart.

5. Vektor, umfassend die isolierte Nucleinsäure nach Anspruch 4.

## Revendications

1. Un ou plusieurs antigène(s) destiné(s) à être utilisé(s) dans un procédé d'induction d'une réponse immunitaire chez une espèce aviaire, dans le(s)quel(s) lesdits un ou plusieurs antigènes comprennent SEQ ID NO:1, ou sont identiques à au moins 99 % à SEQ ID NO:1.

2. Antigène ou composition purifié(e) ou recombinant(e) destiné(e) à être utilisé(e) au niveau d'un procédé d'induction d'une réponse immunitaire chez une espèce aviaire, l'antigène ou la composition comprenant un ou plusieurs antigène(s) *D. gallinae* comprenant la séquence :
(a) SEQ ID NO:1 ;
ou
(b) une séquence au moins à 99 % identique à (a).

3. Composition immunogène comprenant un antigène purifié ou recombinant *D. gallinae* comprenant la séquence :
(a) SEQ ID NO:1 ;
ou
(b) une séquence au moins à 99 % identique à (a).

4. Acide nucléique isolé codant la séquence :
(a) SEQ ID NO:1 ;
ou
(b) une séquence au moins à 99 % identique à (a),
destiné à être utilisé au niveau d'un procédé d'induction d'une réponse immunitaire chez une espèce aviaire.

5. Vecteur comprenant l'acide nucléique isolé selon la revendication 4.
